# EUROPÄISCHE PATENTSCHRIFT

(11) **EP 3 231 505 B1**
(45) Veröffentlichungstag und Bekanntmachung des Hinweises auf die Patenterteilung: **03.10.2018**
(21) Anmeldenummer: 17165049.2
(22) Anmeldetag: 05.04.2017
(51) Int. Cl.: B01F 11/00, B01F 13/00, B01F 15/02

(54) **VORRICHTUNG ZUM MISCHEN UND LAGERN VON POLYMETHYLMETHACRYLAT-KNOCHENZEMENT MIT DRUCKPUMPE UND AMPULLENBRECHER**
DEVICE FOR MIXING AND STORING POLYMETHYL METHACRYLATE BONE CEMENT WITH PRESSURE PUMP AND AMPOULE BREAKER
DISPOSITIF DE MÉLANGE ET DE STOCKAGE DE CIMENT OSSEUX EN POLYMÉTHACRYLATE DE MÉTHYLE COMPRENANT UNE POMPE DE REFOULEMENT ET DISPOSITIF D'OUVERTURE D'AMPOULES

(30) Priorität: 06.04.2016 DE 102016106261
(43) Veröffentlichungstag der Anmeldung: 18.10.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- EP-A1- 2 987 765
- WO-A1-2011/109915
- DE-B3-102014 108 569
- DE-T2- 60 012 383

## Beschreibung

Die Erfindung betrifft eine Vorrichtung zum Mischen von Polymethylmethacrylat-Knochenzement und zum Lagern der Ausgangskomponenten des Knochenzements. Insbesondere betrifft die Erfindung eine Vorrichtung zum Mischen von Polymethylmethacrylat-Knochenzement und zum Lagern einer Monomerflüssigkeit und eines Zementpulvers als Ausgangskomponenten des Knochenzements.

Die Erfindung betrifft ferner ein Verfahren zum Mischen eines Knochenzements, insbesondere eines Polymethylmethacrylat-Knochenzements.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig entstehen können beziehungsweise vorhanden sein können, welche die mechanischen Eigenschaften des ausgehärteten Knochenzements negativ beeinflussen können und die somit später eine Destabilisierung des Knochenzements verursachen können.

Zur Vermeidung von Lufteinschlüssen im Knochenzementteig wurden eine Vielzahl von Vakuum-Zementiersystemen vorgeschlagen, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig.

Das Patent DE 10 2009 031 178 B3 offenbart eine gattungsgemäße Mischvorrichtung mit einem zweiteiligen Austragskolben zum Verschluss einer Zementkartusche. Dabei wird eine Kombination aus einem Gas-durchlässigen Sterilisationskolben und einem Gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

In der WO 00/35506 A1 wird eine Vorrichtung vorgeschlagen, bei der Polymethylmethacrylat-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei der Verwendung von Vakuummischsystemen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Ein interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für einen Mischstab oder ein Mischrohr nicht ohne weiteres dauerhaft vakuumdicht ist. Alle bisher bekannten Full-Prepacked-Mischsysteme nutzen Vakuum oder ein Unterdruck, um die Monomerflüssigkeit in das Zementpulver zu überführen.

Auch die DE 10 2014 108 569 B3 offenbart eine Vakuummischvorrichtung für Knochenzement.

Die Aufgabe der Erfindung besteht also darin, die Nachteile des Stands der Technik zu überwinden. Insbesondere sollen die Nachteile der bekannten Vakuummischsysteme mit externer Vakuumquelle überwunden werden. Die Erfindung hat dabei unter anderem die Aufgabe, eine einfache, geschlossene Vorrichtung zu entwickeln, bei der Polymethylmethacrylat-Knochenzementpulver (Zementpulver) und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Vorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit soll möglichst ausgeschlossen sein. Bei der zu entwickelnden Vorrichtung handelt es sich um ein Full-Prepacked-Mischsystem. Die Vorrichtung soll so beschaffen sein, dass ein Transfer der Monomerflüssigkeit in das Polymethylmethacrylat-Knochenzementpulver ohne die Verwendung von durch Druckluft oder Kompressoren angetriebenen externen Vakuumpumpen, transferiert werden kann. Wichtig ist ferner, dass die Vorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Die Vorrichtung soll ohne zusätzliche technische Ausrüstung autonom verwendungsfähig sein. Weiterhin soll die Vorrichtung in einer zweiten Betriebsweise einen Monomertransfer und eine Vermischung der Zementkomponenten auch unter Verwendung von extern erzeugtem Vakuum zulassen.

Die Vorrichtung soll dahingehend vereinfacht sein, dass mit nur einem manuellen Bedienelement zuerst der Monomerflüssigkeitsbehälter beziehungsweise die Glasampulle geöffnet werden kann und dass erst danach mit dem gleichen Bedienelement, ohne Nutzung von extern erzeugtem Vakuum, ein Transfer der Monomerflüssigkeit in die Kartusche mit darin enthaltenem Zementpulver manuell vorgenommen werden kann. Dadurch soll gewährleistet sein, dass der medizinische Anwender immer zuerst den Monomerflüssigkeitsbehälter öffnet und erst danach ein manuell bewirkter Monomertransfer oder auch in der zweiten Betriebsweise durch externes Vakuum bewirkter Monomertransfer möglich ist. Eine Fehlbedienung soll möglichst konstruktiv ausgeschlossen werden.

Weiterhin hat die Erfindung die Aufgabe, eine Vorrichtung bereit zu stellen, bei der es möglich ist, dass Volumen an Monomerflüssigkeit, die in das Zementpulver transferiert wird, gezielt zu steuern, so dass das Verhältnis des Volumens an Monomerflüssigkeit zur Zementpulvermenge variiert werden kann, um die Konsistenz und damit die Verarbeitungseigenschaften des Knochenzements zu steuern.

Es soll ferner ein Verfahren bereitgestellt werden, das einen Monomertransfer und ein Mischen in Full-Prepacked-Mischsystemen ermöglicht und bei dem nur ein einziges Bedienelement bedient werden muss, um sowohl den Monomerbehälter zu öffnen, als auch die Monomerflüssigkeit zu transferieren. Dabei soll das zu entwickelnde Mischsystem, hauptsächlich aus kostengünstigem Kunststoff gefertigt werden können.

Des Weiteren soll eine kostengünstig zu fertigende und zuverlässig funktionierende Vorrichtung zum Mischen eines medizinischen Zements und gegebenenfalls zur Lagerung der Ausgangskomponenten des Zements sowie ein Verfahren zum Mischen des Knochenzements gefunden werden, bei dem eine möglichst einfache manuelle Bedienung zum Mischen der Ausgangskomponenten angewendet werden kann, möglichst ohne dass eine externe oder zusätzliche Energiequelle verwendet werden muss und ohne dass Lufteinschlüsse in dem Mischgut entstehen.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in dem Full-Prepacked-Mischsystem getrennt gelagert werden können und durch Anwendung der Vorrichtung sicher zusammengeführt werden können.

Die Aufgaben der Erfindung werden gelöst durch eine Vorrichtung zum Mischen von Polymethylmethacrylat-Knochenzement und zum Lagern der Ausgangskomponenten des Knochenzements, insbesondere einer Monomerflüssigkeit und eines Zementpulvers als Ausgangskomponenten des Knochenzements, die Vorrichtung aufweisend eine Kartusche mit einem Innenraum zum Mischen des Knochenzements, wobei der Innenraum auf einer Seite durch einen beweglichen Austragskolben verschlossen ist, eine Aufnahme mit zumindest bereichsweise geschlossenen Seitenwänden zur Aufnahme eines Monomerflüssigkeitsbehälters, wobei die Aufnahme zumindest eine verformbare geschlossene Seitenwand aufweist,
ein Sieb und/oder ein Filter das, der oder die unterhalb der Aufnahme angeordnet ist oder sind, so dass der Inhalt des geöffneten Monomerflüssigkeitsbehälters durch das Sieb und/oder den Filter fließt,
eine Verbindungsleitung, durch die die Monomerflüssigkeit in den Innenraum der Kartusche zu leiten ist,
einen Hohlzylinder, der mit der Verbindungsleitung verbunden ist und der über eine Fluidverbindung mit der Aufnahme verbunden ist, so dass der Hohlzylinder in einer Fluidleitung zwischen der Aufnahme und dem Innenraum der Kartusche angeordnet ist, wobei in dem Hohlzylinder ein axial im Hohlzylinder verschiebbarer Pumpkolben angeordnet ist, und
eine Öffnungseinrichtung, die beweglich gegen die verformbare Seitenwand der Aufnahme gelagert ist, wobei die Öffnungseinrichtung sich bis in den Hohlzylinder erstreckt, wobei die Öffnungseinrichtung durch Eindrücken des Pumpkolbens in den Hohlzylinder gegen die verformbare Seitenwand der Aufnahme zu drücken ist, so dass sich die verformbare Seitenwand derart verformt, dass ein in der Aufnahme befindlicher Monomerflüssigkeitsbehälter durch den Druck der Öffnungseinrichtung zu öffnen ist.

Dass der Hohlzylinder zwischen der Aufnahme für den Monomerflüssigkeitsbehälter, insbesondere für die Glasampulle (enthaltend die Monomerflüssigkeit), und der Kartusche angeordnet ist, bedeutet nicht, dass der Hohlzylinder geometrisch dazwischen angeordnet sein muss, sondern dass der Hohlzylinder bezüglich der Fluidleitung, also der Flussrichtung der Monomerflüssigkeit, wenn diese aus dem geöffneten Monomerflüssigkeitsbehälter in Richtung der Kartusche fließt beziehungsweise gepumpt wird, zwischen der Aufnahme und der Kartusche angeordnet ist.

Damit die Monomerflüssigkeit aus dem geöffneten Monomerflüssigkeitsbehälter (insbesondere der Glasampulle) in den Hohlzylinder fließt, ist die Aufnahme hierzu mit dem Hohlzylinder über die Fluidverbindung verbunden, vorzugsweise über eine Einmündung mit dem Hohlzylinder verbunden.

Damit die Monomerflüssigkeit ohne zusätzliche Krafteinwirkung fließen kann, muss die Vorrichtung bestimmungsgemäß aufgestellt werden, damit die Gravitation die gewünschte Flussrichtung bewirkt. Demzufolge sind die im Rahmen der vorliegenden Erfindung verwendeten Begriffe "oben" und "unten" sowie "oberhalb" und "unterhalb" und "höchsten" und "tiefsten" immer in Bezug auf die bestimmungsgemäße Aufstellung der Vorrichtung bezogen.

Der Innenraum der Kartusche hat vorzugsweise eine zylindrische Geometrie. Die zylindrische Form ist die einfachste, mit der sich der Innenraum der Kartusche und der Hohlzylinder realisieren lassen. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums kann also ein Zylinder mit beliebiger Grundfläche sein und der Mantel des Hohlzylinders kann ein Zylinder mit beliebiger Grundfläche sein, das heißt auch mit nicht kreisförmiger oder runder Grundfläche. Erfindungsgemäß wird jedoch eine zylindrische Geometrie mit rotationssymmetrischer und insbesondere kreisrunder Grundfläche bevorzugt, da diese am einfachsten zu fertigen sind.

Es kann erfindungsgemäß vorgesehen sein, dass gegenüber der verformbaren Seitenwand ein Stützelement vorgesehen ist. Damit wird sichergestellt, dass die mechanische Kraft, die mit der Öffnungseinrichtung auf die verformbare Seitenwand ausgeübt werden kann derart auf den Monomerflüssigkeitsbehälter, vorzugsweise die Glasampulle, wirken kann, dass dieser aufgebrochen oder aufgestochen oder aufgerissen oder geöffnet wird und nicht ausweicht oder weggedrückt wird.

Besonders bevorzugt mündet die Fluidverbindung in der Mantelfläche des Hohlzylinders in den Hohlzylinder.

Als Monomerflüssigkeitsbehälter wird erfindungsgemäß bevorzugt eine Glasampulle verwendet. Glasampullen eignen sich besonders als Monomerflüssigkeitsbehälter, da die Monomerflüssigkeit darin besonders lange gelagert werden kann.

Es kann auch ein Monomerflüssigkeitsbehälter mit mehreren Kammern zur Aufbewahrung der Monomerflüssigkeit verwendet werden. Unter einem Monomerflüssigkeitsbehälter wird im Rahmen der vorliegenden Erfindung also auch eine Vielzahl von separaten einzelnen Teil-Monomerflüssigkeitsbehältern verstanden, die in die Aufnahme eingebracht werden können und die mit der Öffnungseinrichtung durch verformen der verformbaren Seitenwand der Aufnahme geöffnet werden können.

Bei erfindungsgemäßen Vorrichtungen kann vorgesehen sein, dass die Monomerflüssigkeit aus dem geöffneten Monomerflüssigkeitsbehälter in den Hohlzylinder fließen kann und die Verbindungsleitung den Hohlzylinder mit dem Innenraum der Kartusche derart verbindet, dass mit dem Pumpkolben Monomerflüssigkeit aus dem Hohlzylinder durch Eindrücken des Pumpkolbens in den Hohlzylinder durch die Verbindungsleitung in den Innenraum der Kartusche zu drücken ist.

Hierdurch wird erreicht, dass der Pumpkolben nach dem Öffnen des Monomerflüssigkeitsbehälters gleichzeitig zum Übertragen der Monomerflüssigkeit in den Innenraum der Kartusche verwendet werden kann, beziehungsweise als Antrieb zum Einpressen der Monomerflüssigkeit in den Innenraum der Kartusche verwendet werden kann. Der Pumpkolben kann dabei manuell oder über einen internen Energiespeicher, insbesondere eine gespannte Druckfeder, angetrieben werden.

Um eine gute Pumpwirkung zu erreichen und einen Austritt von Monomerflüssigkeit aus dem Pumpkolben zu vermeiden, schließt der Pumpkolben fluiddicht mit den Innenwänden des Hohlzylinders ab. Hierzu kann zumindest eine umlaufende Dichtung vorgesehen sein, die den Pumpkolben mit den Innenwänden des Hohlzylinders abschließt. Der Pumpkolben ist zumindest im Bereich des vorgesehenen Hubs bereichsweise zylindrisch geformt und bildet ein Negativ des Hohlzylinders.

Der Polymethylmethacrylat-Knochenzement wird bevorzugt aus zumindest zwei Komponenten gemischt beziehungsweise ist aus zumindest zwei Komponenten herstellbar. Besonders bevorzugt ist eine Komponente flüssig (die Monomerflüssigkeit) und die andere Komponente pulverförmig (das Zementpulver).

Die Ausgangskomponenten für das Mischgut, insbesondere für den PMMA-Knochenzement, sind erfindungsgemäß bereits in der Kartusche und in dem Monomerflüssigkeitsbehälter, vorzugsweise in einer Glasampulle als Monomerflüssigkeitsbehälter, enthalten, wobei der Monomerflüssigkeitsbehälter besonders bevorzugt in der Aufnahme angeordnet ist. Die Vorrichtung ist erfindungsgemäß bevorzugt auch zum Lagern der Ausgangskomponenten geeignet, insbesondere dann, wenn der Monomerflüssigkeitsbehälter enthaltend die Monomerflüssigkeit bereits in die Vorrichtung eingesetzt ist.

Des Weiteren kann erfindungsgemäße vorgesehen sein, dass in der Kartusche eine Mischeinrichtung angeordnet ist, die von außen bedienbar ist, wobei vorzugsweise die Mischeinrichtung über einen Mischstab bedienbar ist, der durch eine Durchführung in dem Austragskolben ins Innere der Kartusche geführt und beweglich gelagert ist.

Besonders bevorzugt ist der Mischstab in der Durchführung drehbar und in Längsrichtung verschiebbar gelagert. Mit der Mischeinrichtung kann der Inhalt des Innenraums der Kartusche bequem mit dem Mischstab durchmischt werden. Bei der Verwendung von niedrig-viskosen Knochenzementen kann auf die Verwendung eines Mischstabs und einer Mischeinrichtung verzichtet werden, weil die Monomerflüssigkeit vor dem Anquellen des Zementpulvers die Luft der Porenräume zwischen den Zementpulverpartikeln verdrängt hat und die Zementpulverpartikel benetzt hat.

Es kann im Rahmen der vorliegenden Erfindung vorgesehen sein, dass der Austragskolben für Pulver undurchlässig ist, wobei bevorzugt in dem Austragskolben ein Porenfilter angeordnet ist, der für Gas durchlässig ist und für Pulver undurchlässig ist.

Der Porenfilter kann bevorzugt als Porenscheibe ausgebildet sein. Durch die Undurchlässigkeit für Pulver kann verhindert werden, dass das Zementpulver aus dem Inneren der Kartusche austreten kann. Wenn der Austragskolben gasdurchlässig ist, kann der Innenraum durch den Austragskolben evakuiert und mit einem Gas, wie beispielsweise Ethylenoxid, sterilisiert werden.

Gemäß einer bevorzugten Weiterbildung der vorliegenden Erfindung kann vorgesehen sein, dass das Zementpulver im Innenraum der Kartusche enthalten ist.

Es kann auch vorgesehen sein, dass die Monomerflüssigkeit in dem Monomerflüssigkeitsbehälter in der Aufnahme enthalten ist. Hierdurch bildet die Vorrichtung ein fertiges Full-Prepacked-Mischsystem, das nicht vor der Anwendung mit dem Zementpulver gefüllt werden muss. In der Kartusche wird das Zementpulver vor der Verwendung separat zur Monomerflüssigkeit in dem Monomerflüssigkeitsbehälter gelagert.

Ferner kann vorgesehen sein, dass zwischen der Verbindungsleitung und dem Innenraum der Kartusche ein für das Zementpulver undurchlässiger und für die Monomerflüssigkeit durchlässiger Filter angeordnet ist.

Hierdurch kann verhindert werden, dass das Zementpulver in die Verbindungsleitung eindringt und dort beim Einleiten der Monomerflüssigkeit polymerisiert und so ungewollt die Verbindungsleitung verstopft beziehungsweise verklebt.

Gemäß einer Weiterbildung kann auch vorgesehen sein, dass die Vorrichtung einen Standfuß aufweist, in dem zumindest ein Teil der Verbindungsleitung angeordnet ist, wobei die Kartusche lösbar mit dem Standfuß verbunden ist, insbesondere über ein Schraubgewinde lösbar mit dem Standfuß verbunden ist, wobei bevorzugt gegebenenfalls der für das Zementpulver undurchlässige und für die Monomerflüssigkeit durchlässige Filter in dem Standfuß der Vorrichtung angeordnet ist, besonders bevorzugt in der Verbindung zur Kartusche des Standfußes angeordnet ist.

Hierdurch kann die Vorrichtung leicht aufgestellt und einfach bedient werden.

Dabei kann vorgesehen sein, dass der Hohlzylinder und die Aufnahme mit dem Standfuß verbunden sind, bevorzugt unlösbar und/oder einteilig mit dem Standfuß verbunden sind. Hierdurch wird ein besonders einfacher und kostengünstiger Aufbau der Vorrichtung ermöglicht.

Des Weiteren kann vorgesehen sein, dass die Aufnahme an einer Mantelfläche des Hohlzylinders in den Hohlzylinder mündet.

Hierdurch kann sichergestellt werden, dass die Monomerflüssigkeit vollständig in den Hohlzylinder fließen kann und den Hohlzylinder füllen kann. Zudem kann so Luft an dieser Stelle besonders leicht aus dem Hohlzylinder austreten.

Es kann vorgesehen sein, dass in oder an der Fluidverbindung zum Hohlzylinder ein Sieb oder ein Filter angeordnet ist, mit dem Bruchstücke des geöffneten Monomerflüssigkeitsbehälters zurückgehalten werden können.

Mit einer Weiterbildung der Erfindung zur Ausnutzung der Gravitation als Antrieb für den Fluss der Monomerflüssigkeit in den Hohlzylinder wird vorgeschlagen, dass die Aufnahme für den Monomerflüssigkeitsbehälter oberhalb der der Fluidverbindung zum Hohlzylinder angeordnet ist.

Dadurch kann die Monomerflüssigkeit aus dem Monomer-Behälter nach dem Öffnen des Monomerflüssigkeitsbehälters aufgrund der Gravitation durch die Fluidverbindung in den Hohlzylinder fließen.

Bevorzugte erfindungsgemäße Vorrichtungen können sich auch dadurch auszeichnen, dass die Verbindungsleitung an der unteren Seite mit dem Hohlzylinder verbunden ist, bevorzugt am tiefsten Punkt des Hohlzylinders mit dem Hohlzylinder verbunden ist, wobei besonders bevorzugt der Pumpkolben auf der gegenüberliegenden Seite des Hohlzylinders angeordnet ist.

Hierdurch kann die Monomerflüssigkeit mit dem Pumpkolben vollständig aus dem Hohlzylinder abfließen beziehungsweise herausgedrückt werden.

Ferner kann vorgesehen sein, dass der Hohlzylinder auf der dem Pumpkolben gegenüberliegenden Seite einen kegelförmigen, halbkugelförmigen oder anderen sich nach unten verjüngenden Boden aufweist, wobei bevorzugt die dem Boden des Hohlzylinders zugewandte Fläche des Pumpkolbens eine Negativform des Bodens bildet.

Hierdurch kann die Monomerflüssigkeit mit dem Pumpkolben vollständig aus dem Hohlzylinder abfließen beziehungsweise herausgedrückt werden. Das bedeutet, dass die gesamte Monomerflüssigkeit zum tiefsten Punkt des Hohlzylinders fließt und keine toten Bereiche vorhanden sind, in denen Monomerflüssigkeit bei Betätigung des Pumpkolbens zurückbleibt. Durch die Anpassung der Form des Pumpkolbens an die innere Form des Hohlzylinders wird die gesamte Monomerflüssigkeit aus dem Hohlzylinder durch den Pumpkolben bei Bewegung des Pumpkolbens in Richtung der Öffnung zur Verbindungsleitung gepresst, ohne dass Rückstände der Monomerflüssigkeit im Hohlzylinder verbleiben. Weiterhin wird durch diese kegelförmige oder kugelförmige beziehungsweise passende Gestalt der Stirnseite des Pumpkolbens gewährleistet, dass bei Bewegung des Pumpkolbens nach unten beziehungsweise in Richtung des Standfußes, die Luft über der Monomerflüssigkeit im Hohlzylinder durch die Fluidverbindung in der Mantelfläche des Hohlzylinders entweichen kann und keine Luftblasen oberhalb der Monomerflüssigkeit beim Transferieren der Monomerflüssigkeit in den Innenraum der Kartusche beziehungsweise in das Zementpulver verbleiben.

Bevorzugte Ausführungsformen können vorsehen, dass der Pumpkolben manuell axial im Hohlzylinder bewegt werden kann, bevorzugt manuell axial in den Hohlzylinder eingepresst werden kann.

Damit ist es möglich, die Monomerflüssigkeit manuell aus dem Hohlzylinder auszupressen und in den Innenraum der Kartusche zu überführen.

Zur Vereinfachung der Bedienung und für eine höhere Variabilität erfindungsgemäßer Vorrichtungen kann auch vorgesehen sein, dass der Hohlzylinder transparent ist und Markierungen zur Füllstandsmenge einer Flüssigkeit in dem Hohlzylinder aufweist.

Dadurch kann eine durch die Markierungen bestimmte Menge der Monomerflüssigkeit in den Hohlzylinder gefüllt werden beziehungsweise aus dem Hohlzylinder in den Innenraum der Kartusche eingepresst werden. So ergibt sich die Möglichkeit mit der Vorrichtung einen Knochenzementteig mit einer durch die Menge der Monomerflüssigkeit vorgegebenen Konsistenz herzustellen. Alternativ kann der Hohlzylinder auch nicht transparent sein und Markierungen an dem aus dem Hohlzylinder ragenden Ende des Pumpkolbens vorgesehen sein, um einen definierten Vortrieb des Pumpkolbens zu ermöglichen und damit ein definiertes Volumen der Monomerflüssigkeit aus dem Hohlzylinder pressen zu können. Mit derartigen Aufbauten ist es also möglich, sowohl das gesamte Volumen der Monomerflüssigkeit aus dem Hohlzylinder in das Zementpulver im Innenraum der Kartusche zu pressen als auch nur bestimmte Teilvolumen der Monomerflüssigkeit aus dem Hohlzylinder in das Zementpulver zu transferieren. Dadurch kann das Verhältnis von Monomerflüssigkeit zur Pulvermenge eingestellt werden, wodurch das zeitliche Erreichen der Klebfreiheit des gebildeten Zementteigs und die Viskosität des Knochenzements gezielt gesteuert werden kann.

Mit einer Weiterbildung der vorliegenden Erfindung wird vorgeschlagen, dass die Vorrichtung eine gespannte Druckfeder und zumindest eine Arretierung aufweist, wobei die Druckfeder und/oder der Pumpkolben mit der Arretierung lösbar arretiert ist oder sind, wobei bei gelöster Arretierung die Druckfeder einen Druck auf den Pumpkolben ausübt, so dass der Pumpkolben in den Hohlzylinder gepresst wird.

Diese Maßnahmen haben den Vorteil, dass die Bedienung der Vorrichtung vereinfacht wird. Zudem können so mögliche Fehlbedienungen verhindert werden. Die Arretierung kann durch Spangen oder Sicherungsstifte als Rastmittel beziehungsweise Rastvorrichtungen realisiert werden.

Ferner kann vorgesehen sein, dass der Austragskolben mit einer lösbaren Rasteinrichtung mit der Kartusche verbunden ist, wobei die Rasteinrichtung manuell, insbesondere durch axiale Krafteinwirkung lösbar ist, so dass der Austragskolben axial im Innenraum der Kartusche bewegt werden kann.

Hierdurch kann eine ungewollte Bewegung des Austragskolbens, wie sie beispielsweise durch ein Vakuum im Innenraum der Kartusche verursacht werden kann, verhindert werden.

Es kann auch alternativ vorgesehen sein, dass der Hohlzylinder ein Innengewinde aufweist und der Pumpkolben ein dazu passendes Außengewinde aufweist, so dass der Pumpkolben in den Hohlzylinder einschraubbar ist, um die Öffnungseinrichtung gegen die verformbare Seitenwand zu drücken und die Monomerflüssigkeit aus dem Hohlzylinder in den Innenraum der Kartusche zu pressen.

Auch hierdurch kann eine definierte Menge der Monomerflüssigkeit aus dem Hohlzylinder in den Innenraum der Kartusche eingepresst werden. Dadurch ergibt sich die Möglichkeit mit der Vorrichtung einen Knochenzementteig mit einer durch die Menge der Monomerflüssigkeit bestimmten Konsistenz herzustellen.

Zur Vermeidung einer ungewollten Befüllung des Innenraums der Kartusche mit Monomerflüssigkeit kann vorgesehen sein, dass die Verbindungsleitung zwischen dem Hohlzylinder und dem Innenraum der Kartusche eine nach oben weisende Schlaufe aufweist, wobei der höchste Punkt der Schlaufe oberhalb der Einmündung der Aufnahme in den Hohlzylinder liegt.

Damit kann verhindert werden, dass die Monomerflüssigkeit beim Einfüllen in den Hohlzylinder bereits durch die Verbindungsleitung in den Innenraum der Kartusche gelangt. Diese umgekehrt U-förmige Schlaufe der Verbindungsleitung erreicht, dass vor Bewegung des Pumpkolbens in Richtung der Verbindungsleitung zur Kartusche die Monomerflüssigkeit im Hohlzylinder bis zur Höhe des Scheitelpunkts in der Verbindungsleitung verbleibt, wodurch ein vorzeitiger Eintritt der Monomerflüssigkeit zum Zementpulver verhindert wird. Insbesondere bei hoch-viskosen Zementen kann ein vorzeitiger Kontakt von schon geringen Mengen der Monomerflüssigkeit mit dem Zementpulver zur Verklebung der Verbindungsleitung oder eines als Düse ausgebildeten Leitungsmittels, wie in US 8 662 736 B2 beschrieben, führen. Die Verbindungsleitung kann transparent oder transluzent sein, damit der Anwender den Monomertransfer visuell kontrollieren kann. Hierzu kann insbesondere ein Sichtfenster in der Vorrichtung vorgesehen sein, durch das die Schlaufe mit dem höchsten Scheitelpunkt zu erkennen ist.

Des Weiteren kann erfindungsgemäß vorgesehen sein, dass das Volumen im Hohlzylinder kleiner oder gleich dem Volumen der Monomerflüssigkeit in dem Monomerflüssigkeitsbehälter ist.

Dadurch wird verhindert, dass bei Betätigung des Pumpkolbens Luft mit in das Zementpulver gepresst werden kann.

Ferner kann vorgesehen sein, dass der Innenraum der Kartusche an der unteren Seite mit der Verbindungsleitung flüssigkeitsdurchlässig verbunden ist.

Die Verbindungsleitung kann in der Stirnseite des Innenraums in eine Düse gemäß US 8 662 736 B4 münden. Diese Düse verhindert einen Zutritt von Zementpulver in das Leitungsmittel.

In einer anderen Ausgestaltungsvariante ist der Innenraum der Kartusche an einer seitlichen Mantelfläche mit der Verbindungsleitung flüssigkeitsdurchlässig verbunden. Es ist damit auch möglich, die Monomerflüssigkeit seitlich in das Zementpulver in den Innenraum der Kartusche zu transferieren.

Bevorzugt bestehen der Hohlzylinder, die Kartusche und die Verbindungsleitung sowie, sofern vorhanden, auch der Standfuß, die Mischeinrichtung und der Mischstab aus einem Kunststoff und können durch Kunststoffspritzgießen kostengünstig hergestellt werden.

Bevorzugt kann auch vorgesehen sein, dass die Öffnungseinrichtung ein Hebel ist, der um eine Achse drehbar gegen die Aufnahme gelagert ist, wobei ein freies Ende des Hebels von dem Pumpkolben gegen die verformbare Seitenwand der Aufnahme drückbar ist, so dass das freie Ende des Hebels die verformbare Seitenwand derart verformt, dass ein in der Aufnahme befindlicher und zur Aufnahme passender Monomerflüssigkeitsbehälter durch den Druck des freien Endes des Hebels zu öffnen ist, insbesondere aufzubrechen oder aufzuschlitzen oder aufzustechen ist.

Hierdurch wird eine besonders einfache, kostengünstig zu realisierende und dabei fehlerunanfällige Öffnungseinrichtung bereitgestellt, die leicht durch die Bewegung des Pumpkolbens aktiviert werden kann.

Dabei kann vorgesehen sein, dass das freie Ende mit dem Hebel derart im Inneren des Hohlzylinders angeordnet ist und an der verformbaren Seitenwand anliegt, dass der Hebel ausgehend von der Achse mit dem freien Ende und der verformbaren Seitenwand ein Dreieck mit einem Steg als Schenkel gegenüber der Ecke, die durch die Drehachse gebildet ist, bildet, wobei das Dreieck im Hohlzylinder angeordnet ist.

Hiermit wird erreicht, dass das Dreieck durch axiale Bewegung des Pumpkolbens um die Drehachse geschwenkt werden kann und so die verformbare Seitenwand zur Öffnung des Monomerflüssigkeitsbehälters in der Aufnahme mit dem freien Ende verformt wird.

Dabei kann vorgesehen sein, dass das Verhältnis der Länge des Hebels, ausgehend vom Drehpunkt der Achse bis zum Ansatz des Stegs, zur Länge des Stegs vom Ansatzpunkt des Stegs auf dem Hebel bis zur Spitze des freien Endes, mindestens 3 zu 1 ist, bevorzugt 4 zu 1 und ganz besonders bevorzugt größer 5 zu 1 ist.

Bevorzugt kann dabei vorgesehen sein, dass der Hebel an einer Längsseite einer fensterförmigen Öffnung oberhalb der Fluidverbindung drehbar mit einem Scharnier so angeordnet wird, das die Drehachse des Hebels senkrecht zur Längsachse des Hohlzylinders steht, wobei das Scharnier außerhalb des Hohlzylinders an dessen äußerer Mantelfläche angeordnet ist.

Des Weiteren kann vorgesehen sein, dass das freie Ende des Hebels als Keil ausgeformt ist, der an der verformbaren Seitenwand der Aufnahme anliegt. Besonders bevorzugt weist der Hebel einen Steg auf, der senkrecht zur Drehachse des Scharniers ausgerichtet ist, wobei ganz besonders bevorzugt das freie Ende des Hebels das eine Ende des Stegs begrenzt.

Mit einer Weiterbildung der vorliegenden Erfindung wird vorgeschlagen, dass die Aufnahme ein Hohlzylinder ist und/oder die Aufnahme aus einem Elastomer besteht oder einen Einsatz aus einem Elastomer umfasst, wobei vorzugsweise das Elastomer eine Shore-Härte größer 60 aufweist, wobei besonders bevorzugt das Elastomer ein Silikonkautschuk oder ein Ethylen-Propylen-Dien-Kautschuk (EPDM) ist.

Durch die zylindrische Geometrie der Aufnahme wird erreicht, dass eine zylindrische Glasampulle als Monomerflüssigkeitsbehälter mit festem Sitz in die Aufnahme eingesteckt werden kann. Mit der angegebenen Härte nach Shore wird erreicht, dass die Glasampulle sicher innerhalb der Aufnahme geöffnet werden kann, ohne dass die Aufnahme zerstört wird oder ein Leck entwickelt.

Ferner kann vorgesehen sein, dass in der Aufnahme ein Absatz zur Auflage des Monomerflüssigkeitsbehälters angeordnet ist, wobei der Absatz kleiner als die Hälfte der Fläche des Bodens des Monomerflüssigkeitsbehälters oder des Querschnitts des Monomerflüssigkeitsbehälters ist.

Hierdurch wird der Monomerflüssigkeitsbehälter, insbesondere die Glasampulle, in der Aufnahme fixiert, so dass er beziehungsweise sie in definierter Position mit Hilfe der Öffnungseinrichtung zu öffnen beziehungsweise aufzubrechen ist und nicht ausweichen kann. Der Absatz ist vorzugsweise so in der Aufnahme angeordnet, dass der Abstand zwischen dem Absatz und dem flüssigkeitsdurchlässigen Sieb und/oder Filter gleich oder größer ist als der Durchmesser des Monomerflüssigkeitsbehälters beziehungsweise der Glasampulle.

Es kann auch vorgesehen sein, dass ein freies Ende der Öffnungseinrichtung bei einer Bewegung des Pumpkolbens in den Hohlzylinder hinein derart auf die verformbare Seitenwand drückt, dass der Vektor der Kraft eine Komponente aufweist, die in Richtung des Siebs und/oder Filters gerichtet ist und/oder die einen in die Aufnahme eingesetzter Monomerflüssigkeitsbehälter in die Aufnahme hineindrückt, vorzugsweise in Richtung des Absatzes drückt.

Hierdurch wird erreicht, dass der Monomerflüssigkeitsbehälter beim Bewegen der Öffnungseinrichtung nicht aus der Aufnahme herausgedrückt werden kann und die Positionierung des Monomerflüssigkeitsbehälters sichergestellt ist. Dies führt dazu, dass der Monomerflüssigkeitsbehälter sicher geöffnet wird und nicht durch eine Bewegung aus der Aufnahme hinaus ausweichen kann.

Es kann vorgesehen sein, dass am freien Ende der Öffnungseinrichtung, insbesondere am freien Ende des Hebels, auf der zur Aufnahme zugewandten Seite ein Keil angeordnet ist.

Mit dem Keil wird eine definierte Kraft auf eine kleine Fläche des Monomerflüssigkeitsbehälters ausgeübt, so dass der Monomerflüssigkeitsbehälter mit möglichst geringem Kraftaufwand an einer möglichst genau definierten Stelle aufzubrechen ist. Dieser Keil kann dreieckig oder keilförmig ausgebildet sein. Es ist auch möglich, dass der Keil pyramidenförmig, prismenförmig oder kegelförmig ausgebildet ist.

Ferner kann vorgesehen sein, dass in der Aufnahme ein Monomerflüssigkeitsbehälter, insbesondere eine Glasampulle, angeordnet ist, die eine Flüssigkeit enthält, vorzugsweise eine Monomerflüssigkeit zur Herstellung eines medizinischen Knochenzements enthält.

Hierdurch kann die Vorrichtung unmittelbar zum Öffnen des Monomerflüssigkeitsbehälters beziehungsweise der Glasampulle eingesetzt werden, ohne dass vorher der Monomerflüssigkeitsbehälter beziehungsweise die Glasampulle eingesetzt werden müsste.

Des Weiteren kann erfindungsgemäß vorgesehen sein, dass die Öffnungseinrichtung sich oberhalb der Fluidverbindung in den Hohlzylinder erstreckt.

Hierdurch wird sichergestellt, dass zuerst der Monomerflüssigkeitsbehälter geöffnet wird, ausläuft und die Monomerflüssigkeit aus dem Monomerflüssigkeitsbehälter durch die Fluidverbindung in den Hohlzylinder fließen kann, bevor die Monomerflüssigkeit mit dem Pumpkolben aus dem Hohlzylinder durch die Verbindungsleitung in die Kartusche gedrückt wird.

Mit der vorliegenden Erfindung wird auch vorgeschlagen, dass der Pumpkolben mit einer manuell lösbaren Rastvorrichtung fixierbar ist, die nach axialer Verschiebung des Pumpkolbens über die Öffnungseinrichtung im Hohlzylinder, den Pumpkolben oberhalb der Einmündung der Fluidverbindung zur Aufnahme im Hohlzylinder blockiert, so dass der Pumpkolben im Hohlzylinder nicht über die Einmündung der Fluidverbindung bewegbar ist, wenn die Rastvorrichtung nicht gelöst ist, wobei bevorzugt die Rastvorrichtung am oder im Pumpkolben angeordnet ist oder sich durch den Pumpkolben erstreckt.

Hiermit wird erreicht, dass der Pumpkolben zunächst die Öffnungseinrichtung bedient und dadurch der Monomerflüssigkeitsbehälter in der Aufnahme geöffnet wird und dann von der Rastvorrichtung aufgehalten wird. Bis die Rastvorrichtung gelöst ist, um eine weitere Bewegung des Pumpkolbens in den Hohlzylinder zu ermöglichen, bleibt somit genug Zeit, damit die Monomerflüssigkeit aus dem Monomerflüssigkeitsbehälter in den Hohlzylinder auslaufen kann. Damit wird sichergestellt, dass eine ausreichende Menge der Monomerflüssigkeit in dem Hohlzylinder zur Verfügung steht, bevor diese mit dem Pumpkolben in die Kartusche gepresst wird.

Die der vorliegenden Erfindung zugrundeliegenden Aufgaben werden auch gelöst durch ein Verfahren zum Mischen eines Knochenzements, insbesondere mit einer erfindungsgemäßen Vorrichtung, mit den chronologischen Schritten:
A) ein Pumpkolben wird in einen Hohlzylinder gedrückt, wobei durch die Bewegung des Pumpkolbens eine Öffnungseinrichtung gegen eine verformbare Seitenwand einer Aufnahme gedrückt wird,
B) durch die Verformung der Seitenwand der Aufnahme mit der Öffnungseinrichtung wird in der Aufnahme ein Monomerflüssigkeitsbehälter enthaltend eine Monomerflüssigkeit geöffnet,
C) die Monomerflüssigkeit fließt aus dem geöffneten Monomerflüssigkeitsbehälter aus und in den Hohlzylinder hinein,
D) der Pumpkolben wird weiter in den Hohlzylinder gedrückt und die Monomerflüssigkeit dadurch aus dem Hohlzylinder und durch eine Verbindungsleitung in den Innenraum einer Kartusche eingepresst, wobei sich in dem Innenraum der Kartusche ein Zementpulver befindet, und
E) die Monomerflüssigkeit und das Zementpulver werden in dem Innenraum der Kartusche gemischt.

Bevorzugt fließt die Monomerflüssigkeit durch Einwirkung der Schwerkraft in den Hohlzylinder.

Ebenfalls bevorzugt fließt die Monomerflüssigkeit durch ein Sieb und/oder einen Filter in den Hohlzylinder. Das Sieb und/oder der Filter können Bruchstücke des Monomerflüssigkeitsbehälters, die beim Aufbrechen des Monomerflüssigkeitsbehälters entstehen können, zurückhalten.

Bei erfindungsgemäßen Verfahren kann vorgesehen sein, dass in Schritt A) die Bewegung des Pumpkolbens durch eine Rastvorrichtung gestoppt wird und vor Schritt D) die Rastvorrichtung gelöst wird, um den Pumpkolben weiter in den Hohlzylinder drücken zu können.

Hierdurch wird vermieden, dass der Pumpkolben kurz nach dem Öffnen des Monomerflüssigkeitsbehälters bereits so weit in den Hohlzylinder eingedrückt wird, dass die Monomerflüssigkeit nicht weiter in den Hohlzylinder fließen kann und dadurch nicht in ausreichender Menge im Hohlzylinder zur Verfügung steht.

Ferner kann vorgesehen sein, dass der Pumpkolben von einem gespannten Federelement in den Hohlzylinder gedrückt wird, wobei hierfür vorzugsweise zuvor ein Rastmittel oder eine Arretierung gelöst wird, die in den Pumpkolben und/oder in das Federelement greift.

Hiermit muss die Kraft, die zum Drücken des Pumpkolbens in den Hohlzylinder benötigt wird, nicht manuell aufgebracht werden. Das Verfahren wird hierdurch weiter automatisiert.

Des Weiteren kann vorgesehen sein, dass der Pumpkolben vor Schritt D) mit einer Rastvorrichtung oder einer Arretierung gegen den Hohlzylinder gesichert ist, wobei die Rastvorrichtung oder Arretierung gelöst wird, bevor der Pumpkolben in Schritt D) weiter in den Hohlzylinder gedrückt wird.

Hierdurch wird eine ungewollte Bewegung des Pumpkolbens in den Hohlzylinder hinein vermieden.

Es kann erfindungsgemäß vorgesehen sein, dass die Monomerflüssigkeit und das Zementpulver erst dann in dem Innenraum der Kartusche gemischt werden, wenn der Pumpkolben vollständig oder bis zu einer Markierung in den Hohlzylinder eingedrückt wurde, wobei die Markierung ein Maß für die in den Innenraum der Kartusche eingeleitete Monomerflüssigkeit ist.

Hierdurch kann sichergestellt werden, dass der erzeugte Knochenzementteig die gewünschte Konsistenz durch die gewünschte Beimengung von Monomerflüssigkeit erhält.

Mit einer Weiterbildung des erfindungsgemäßen Verfahrens wird vorgeschlagen, dass die Monomerflüssigkeit und das Zementpulver in dem Innenraum mit einer Mischeinrichtung gemischt werden, indem die Mischeinrichtung durch Bewegen eines drehbar und in Längsrichtung verschiebbar in den Innenraum der Kartusche geführten Mischstabs bedient wird, wobei bevorzugt nach dem Mischen der Mischstab bis zum Anschlag aus dem Innenraum der Kartusche herausgezogen wird und besonders bevorzugt der Mischstab nach dem Herausziehen bis zum Anschlag an einer Sollbruchstelle abgebrochen wird.

Hierdurch kann das Verfahren einfach durch manuelle Bedienung durchgeführt werden.

Es kann auch vorgesehen sein, dass die Kartusche mit dem fertig gemischten Zementteig von der Verbindungsleitung, dem Hohlzylinder und der Aufnahme gelöst wird und der fertig gemischte Zementteig durch Vortreiben eines Austragskolbens, der axial beweglich in der Kartusche gelagert ist und der den Innenraum der Kartusche auf einer Seite begrenzt, aus dem Innenraum der Kartusche ausgetragen wird.

Hiermit kann der Zementteig ohne großen Aufwand angewendet beziehungsweise später bei einem Patienten appliziert werden.

Schließlich kann auch vorgesehen sein, dass die Monomerflüssigkeit zusätzlich durch ein Vakuum im Innenraum der Kartusche in die Kartusche gesaugt wird, wobei vorzugsweise das Vakuum in der Kartusche durch Evakuieren des Innenraums der Kartusche durch den an der Kartusche arretierten Austragskolben erfolgt.

Hierdurch kann der Transfer der Monomerflüssigkeit in die Kartusche unterstützt werden. Zudem kann so die Menge der Lufteinschlüsse beziehungsweise die Gefahr für das Entstehen von Lufteinschlüssen weiter reduziert werden. Das Vakuum kann theoretisch durch die Bewegung des Pumpkolbens erzeugt werden, in dem der Pumpkolben auf seiner Rückseite einen Unterdruck erzeugt, der über den arretierten Austragskolben in den Innenraum der Kartusche geleitet wird.

Der Erfindung liegt die überraschende Erkenntnis zugrunde, dass durch die Bewegung eines Pumpkolbens eine Öffnungseinrichtung bedient werden kann, mit der eine Glasampulle enthaltend eine Monomerflüssigkeit geöffnet werden kann. Ferner wurde überraschend gefunden, dass die Monomerflüssigkeit anschließend mit Hilfe der gleichen Bewegung desselben Pumpkolbens von unten in den Innenraum einer Kartusche eingepresst werden kann, ohne dass sich hierbei störende Lufteinschlüsse im Knochenzement bilden. Dadurch kann mit der Vorrichtung weitgehend auf externe Energiequellen oder interne Energiespeicher verzichtet werden. Eine gespannte Feder kann die Bewegung des Pumpkolbens auslösen beziehungsweise unterstützen. Insbesondere müssen keine Vakuumquellen und vakuumdichten Anschlüsse und Bauteile verwendet werden, was den Einsatz an weniger entwickelten Orten sowie im Einsatz vor Ort oder in Feldlazaretten erheblich erleichtert. Zudem sind erfindungsgemäße Full-Prepacked-Mischsysteme unanfälliger für mögliche Störungen und dadurch mit sehr hoher Wahrscheinlichkeit einsatzbereit, weil keine Vakuumlecks auftreten können.

Der Erfindung basiert ferner auf der überraschenden Erkenntnis, dass es mit der erfindungsgemäßen Vorrichtung gelingt, eine Glasampulle großflächig innerhalb der Vorrichtung beziehungsweise der Zementiervorrichtung aufzubrechen, so dass die Monomerflüssigkeit aus der Glasampulle in kurzer Zeit ausströmt und zum Mischen mit einem medizinischen Knochenzementpulver bereitsteht. Mit Hilfe eines einfachen Hebels als Öffnungseinrichtung ist es möglich, den Druck auf die Glasampulle in Richtung des Sitzes der Glasampulle in der Aufnahme zu richten, so dass ein Ausweichen der Glasampulle aus der Aufnahme heraus ausgeschlossen werden kann. Gleichzeitig kann ein sehr genau definierter lokaler Druck auf die Glasampulle ausgeübt werden, mit dem die Glasampulle in der Vorrichtung aufgebrochen werden kann. Mit Hilfe der verformbaren Seitenwand kann dafür gesorgt werden, dass die Kraft durch diese Seitenwand ins Innere der Aufnahme auf die Glasampulle übertragen wird, wobei die Aufnahme dabei geschlossen bleibt. Ein Austreten der Monomerflüssigkeit aus der Aufnahme kann somit ausgeschlossen werden. Mit Hilfe des Siebs und/oder des Filters können Glassplitter, die beim Öffnen der Glasampulle entstehen können, zurückgehalten werden. Die Monomerflüssigkeit ist dann zum Mischen mit dem Knochenzementpulver nutzbar.

Beispielsweise kann bei einer erfindungsgemäßen Vorrichtung beziehungsweise einem erfindungsgemäßen Verfahren vorgesehen sein, dass nach Öffnung der Glasampulle durch die Bewegung der Öffnungseinrichtung, die von der Bewegung des Pumpkolbens angetrieben wird, die Monomerflüssigkeit durch Schwerkrafteinwirkung in einen Hohlzylinder fließt, aus diesem durch manuelle Betätigung des Pumpkolbens in den Innenraum einer Kartusche gepresst wird, die Zementpulver enthält. Das bedeutet, dass im Gegensatz zu den bisher marktüblichen Mischsystemen der Transfer der Monomerflüssigkeit nicht durch Vakuum sondern durch Druckeinwirkung erfolgt und dass der gleiche Druck en passant oder nebenbei zum Antrieb der Öffnungseinrichtung und damit zum Aufbrechen der Glasampulle verwendet wird, die die Monomerflüssigkeit enthält. Ein solcher manuell zu betätigender Monomertransfer durch Druckeinwirkung kann kostengünstig mit einfachen durch Kunststoffspritzgießen herzustellenden Kunststoffteilen realisiert werden. Der besondere Vorteil der erfindungsgemäßen Vorrichtung besteht darin, dass die Vorrichtung ohne äußere Hilfsmittel, wie durch Druckluft angetriebene Vakuumpumpen, und ohne externe Energiequellen, wie Druckluft oder Strom, betrieben werden kann. Die erfindungsgemäße Vorrichtung ist dadurch autonom verwendbar und kann selbst unter einfachsten Operationsbedingungen verwendet werden. Mit der erfindungsgemäßen Vorrichtung wird ein geschlossenes Full-Prepacked-Mischsystem für preissensitive Märkte zur Verfügung gestellt.

Es wurde im Rahmen der vorliegenden Erfindung gefunden, dass bei der durch Druck verursachten Einleitung der Monomerflüssigkeit in das Zementpulver von der Unterseite des Innenraums der Kartusche die Monomerflüssigkeit in Form einer einheitlichen Front von unten nach oben wandert. Dadurch wird die Luft, die sich in den Zwischenräumen zwischen den Zementpulverpartikeln befindet, verdrängt und nach oben herausgedrückt. Lufteinschlüsse werden dadurch vermieden. Es wurde im Rahmen der vorliegenden Erfindung überraschend gefunden, dass ein mit einer erfindungsgemäßen Vorrichtung und einem erfindungsgemäßen Verfahren hergestellter Knochenzementteig sehr weitgehend frei von Lufteinschlüssen ist und in der Qualität einem unter Vakuum gemischten Zementteig entspricht.

Eine erfindungsgemäße Vorrichtung zur Lagerung und Vermischung von Polymethylmethacrylat-Knochenzement kann beispielsweise aufweisen:
a) einen ersten Hohlzylinder als Kartuscheninnenraum, wobei eine erste Stirnseite des ersten Hohlzylinders mit mindestens einem für Zementpulver undurchlässigen Austragskolben verschlossen ist und wobei die zweite Stirnseite des ersten Hohlzylinders für Zementpulver undurchlässig ist, wodurch ein erster Hohlraum durch den Austragskolben und den durch die zweite Stirnseite begrenzten ersten Hohlzylinder gebildet wird, in dem Zementpulver angeordnet ist,
b) wobei der erste Hohlraum über eine für Zementpulver undurchlässige, flüssigkeitsdurchlässige erste Öffnung mit einem flüssigkeitsdurchlässigen Leitungsmittel mit einem zylinderförmigen zweiten Hohlraum in einem zweiten Hohlzylinder verbunden ist,
c) wobei an der Mantelfläche des zweiten Holzylinders eine für Flüssigkeiten durchlässige zweite Öffnung angeordnet ist, welche den zweiten Hohlraum, mit einem Hohlkörper, der außerhalb des zweiten Hohlzylinders angeordnet ist, der mindestens einen Monomerflüssigkeitsbehälter enthält, flüssigkeitsdurchlässig verbunden ist, wobei der Hohlkörper oberhalb der Öffnung in der Mantelfläche des zweiten Hohlzylinders angeordnet ist,
d) wobei an der Mantelfläche des zweiten Hohlzylinders eine fensterförmige dritte Öffnung oberhalb der zweiten Öffnung angeordnet ist, wobei ein Hebel an einer Längsseite der fensterförmigen dritten Öffnung drehbar mit einem Scharnier so angeordnet wird, das die Drehachse des Hebels senkrecht zur Längsachse des zweiten Hohlzylinders steht,
e) wobei das Scharnier außerhalb des zweiten Hohlraums an der äußeren Mantelfläche des zweiten Hohlzylinders angeordnet ist,
f) wobei am Hebelende gegenüber dem Drehpunkt des Scharniers ein Keil angeordnet ist, dessen Keil nach außen, entgegengesetzt zur Längsachse des zweiten Hohlzylinders, weist,
g) wobei der Keil in Richtung einer deformierbaren Wand ausgerichtet ist, die mindestens einen Teil eines dritten Hohlkörpers bildet, der den mindestens einen Monomerflüssigkeitsbehälter aufnimmt,
h) wobei der Keil mit dem Hebel beim ungeöffneten Monomerflüssigkeitsbehälter im Inneren des zweiten Hohlzylinders so angeordnet ist, das der Hebel ausgehend vom Scharnier mit dem Keil und der deformierbaren Wand ein Dreieck bildet, das zumindest bereichsweise im zweiten Hohlraum angeordnet ist,
i) wobei mindestens ein Teil des Monomerflüssigkeitsbehälters hinter der deformierbaren Wand gegenüber dem Keil des Hebels angeordnet ist,
j) einem im zylinderförmigen zweiten Hohlraum angeordneten Pumpkolben, der oberhalb der fensterförmigen dritten Öffnung des zweiten Hohlzylinders angeordnet ist und
k) wobei der Pumpkolben axial im zweiten Hohlzylinder so verschiebbar ist, dass der Hebel mit dem Keil gegen die deformierbare Wand durch die axiale Bewegung des Pumpkolbens gedrückt werden kann.

Ein beispielhaftes Verfahren, das beispielsweise mit einer solchen beispielhaften Vorrichtung umgesetzt werden kann, kann dadurch realisiert werden, indem
a) durch axiale Verschiebung des Pumpkolbens im zweiten Hohlraum nach unten in Richtung des Fußteils der Hebel nach außen in Richtung der deformierbaren Wand der fensterartigen dritten Öffnung geschwenkt wird, wobei der Hebel um das Scharnier gedreht wird,
b) der Hebel die deformierbare Wand des zweiten Hohlraums nach außen drückt und dadurch den mindestens einen Monomerflüssigkeitsbehälter öffnet,
c) die Monomerflüssigkeit durch Einwirkung der Schwerkraft durch die zweite Öffnung in der Mantelfläche des zweiten Hohlzylinders in den zweiten Hohlraum fließt,
d) nach Transfer der Monomerflüssigkeit aus dem mindestens einen Monomerflüssigkeitsbehälter in den zweiten Hohlraum der Pumpkolben manuell in Richtung des Fußteils der Vorrichtung gedrückt wird,
e) dadurch die Monomerflüssigkeit durch das Leitungsmittel in den ersten Hohlraum in das Zementpulver gedrückt wird,
f) nach Transfer der Monomerflüssigkeit vom zweiten Hohlraum in den ersten Hohlraum das Gemisch aus Zementpulver und Monomerflüssigkeit manuell durch Betätigung eines Mischstabs gemischt wird,
g) dann der Mischstab nach oben gezogen wird und der Mischstab an einer Sollbruchstelle abgebrochen wird und
h) dann der erste Hohlzylinder vom Fußteil getrennt wird.

Alternativ kann auch das Folgende erfindungsgemäße Verfahren mit der beispielhaften Vorrichtung umgesetzt werden:
a) Durch axiale Verschiebung des Pumpkolbens im zweiten Hohlraum nach unten in Richtung des Fußteils wird der Hebel nach außen in Richtung der deformierbaren Wand (der verformbaren Seitenwand) der fensterartigen dritten Öffnung geschwenkt, wobei der Hebel um das Scharnier gedreht wird,
b) der Hebel drückt die deformierbare Wand des zweiten Hohlraums nach außen und öffnet dadurch den mindestens einen Monomerflüssigkeitsbehälter,
c) die Monomerflüssigkeit fließt durch Einwirkung der Schwerkraft durch die zweite Öffnung in der Mantelfläche des zweiten Hohlzylinders in den zweiten Hohlraum,
d) nach Transfer der Monomerflüssigkeit aus dem mindestens einen Monomerflüssigkeitsbehälter in den zweiten Hohlraum an den Austragskolben des ersten Holzylinders wird ein extern erzeugtes Vakuum angelegt, wodurch die Monomerflüssigkeit durch das Leitungsmittel in den ersten Hohlraum in das Zementpulver gesaugt wird,
e) nach Transfer der Monomerflüssigkeit vom zweiten Hohlraum in den ersten Hohlraum wird das Gemisch aus Zementpulver und Monomerflüssigkeit manuell durch Betätigung eines Mischstabs gemischt, und anschließend der Mischstab nach oben gezogen und an einer Sollbruchstelle abgebrochen, und
f) der erste Hohlzylinder wird vom Fußteil getrennt.

Ein weiteres beispielhaftes Verfahren, das mit der Vorrichtung durchgeführt werden kann, ist durch folgende nacheinander ablaufende Schritte gekennzeichnet:
a) durch axiale Verschiebung des Pumpkolbens im zweiten Hohlraum nach unten in Richtung des Fußteils wird der Hebel nach außen in Richtung der deformierbaren Wand der fensterartigen dritten Öffnung geschwenkt, wobei der Hebel um das Scharnier gedreht wird,
b) der Hebel drückt die deformierbare Wand des zweiten Hohlraums nach außen und öffnet dadurch den mindestens einen Monomerflüssigkeitsbehälter,
c) die Monomerflüssigkeit fließt durch Einwirkung der Schwerkraft durch die zweite Öffnung in der Mantelfläche des zweiten Hohlzylinders in den zweiten Hohlraum,
d) nach vollständigen Transfer der Monomerflüssigkeit in den zweiten Hohlraum wird eine Rastvorrichtung des Pumpkolbens gelöst, wobei eine Druckfeder den Pumpkolben in Richtung des Fußteils der Vorrichtung drückt,
e) dadurch die Monomerflüssigkeit durch das Leitungsmittel in den ersten Hohlraum in das Zementpulver gedrückt wird,
f) wobei nach Transfer der Monomerflüssigkeit vom zweiten Hohlraum in den ersten Hohlraum das Gemisch aus Zementpulver und Monomerflüssigkeit manuell durch Betätigung eines Mischstabs gemischt wird, wobei anschließend der Mischstab nach oben gezogen wird und an einer Sollbruchstelle abgebrochen wird und
g) dann der erste Hohlzylinder vom Fußteil getrennt wird.

Im Folgenden werden zwei weitere Ausführungsbeispiele der Erfindung anhand von zwölf schematisch dargestellten Figuren erläutert, ohne jedoch dabei die Erfindung zu beschränken. Dabei zeigt:
- Figur 1:: eine schematische perspektivische Teilschnittansicht einer erfindungsgemäßen Vorrichtung als Full-Prepacked-Mischsystem;
- Figur 2:: die Vorrichtung nach Figur 1 in einer schematischen Seitenansicht;
- Figur 3:: eine schematische Querschnittansicht der erfindungsgemäßen Vorrichtung nach den Figuren 1 und 2 in einer Ausgangssituation;
- Figur 4:: eine schematische Querschnittansicht der erfindungsgemäßen Vorrichtung nach Figur 3, bei der der Pumpkolben ein Stück eingedrückt ist und die Glasampulle geöffnet ist;
- Figur 5:: eine schematische perspektivische Querschnittansicht der erfindungsgemäßen Vorrichtung nach Figur 4;
- Figur 6:: eine schematische Querschnittansicht der erfindungsgemäßen Vorrichtung nach Figur 4 und 5, bei der der Pumpkolben vollständig eingedrückt ist und die Monomerflüssigkeit in die Kartusche gedrückt wurde;
- Figur 7:: eine schematische perspektivische Seitenansicht einer alternativen zweiten erfindungsgemäßen Vorrichtung;
- Figur 8:: die Vorrichtung nach Figur 7 in einer schematischen Seitenansicht gegenüberliegend der Ansicht nach Figur 7;
- Figur 9:: eine schematische Querschnittansicht der zweiten erfindungsgemäßen Vorrichtung nach den Figuren 7 und 8 in einer Ausgangssituation;
- Figur 10:: eine schematische Querschnittansicht eines Ausschnitts der zweiten erfindungsgemäßen Vorrichtung nach Figur 9, bei der der Pumpkolben ein Stück eingedrückt ist und die Glasampulle geöffnet ist;
- Figur 11:: eine schematische Querschnittansicht des Ausschnitts der zweiten erfindungsgemäßen Vorrichtung nach Figur 10, bei der der Pumpkolben vollständig eingedrückt ist und die Monomerflüssigkeit in die Kartusche gedrückt wurde; und
- Figur 12:: eine schematische Querschnittansicht eines Teilbereichs der zweiten erfindungsgemäßen Vorrichtung nach den Figuren 7 bis 11, wobei die Schnittebene parallel zur Spange beziehungsweise dem Rastmittel verläuft.

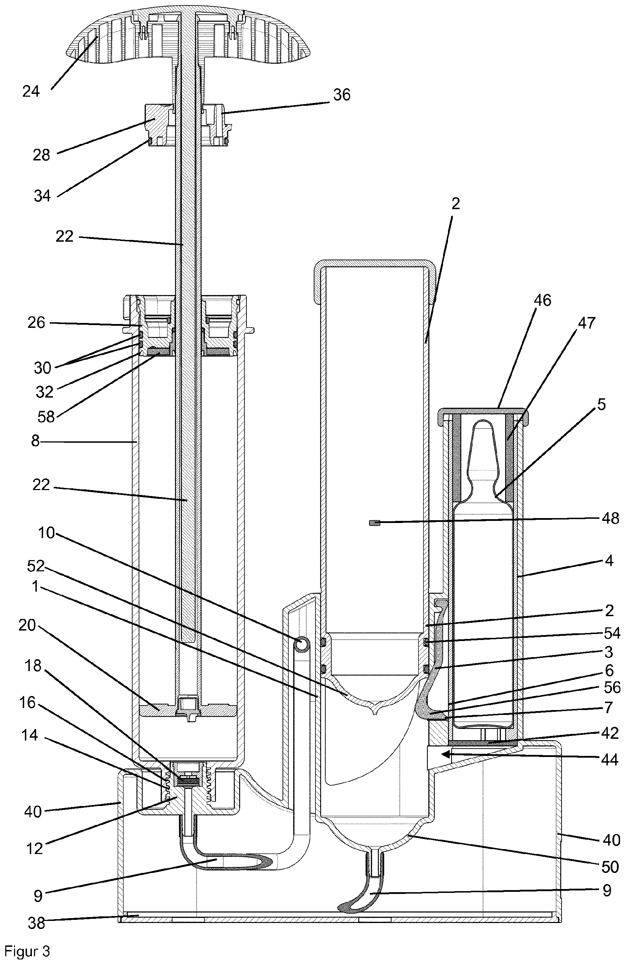

In den Querschnittansichten der Figuren 3, 4, 6, 9, 10, 11 und 12 sind geschnittene Flächen durch Schraffierungen kenntlich gemacht.

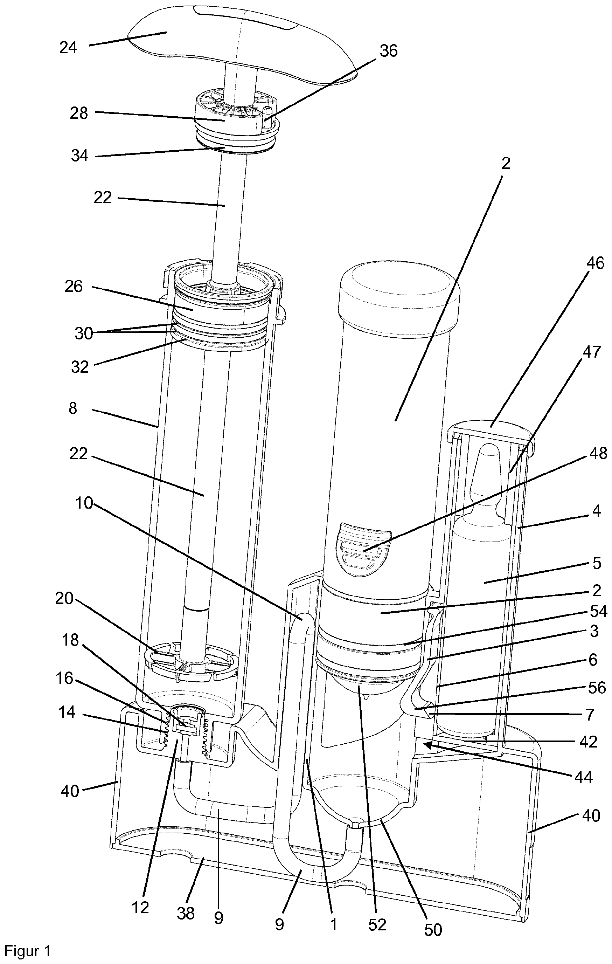

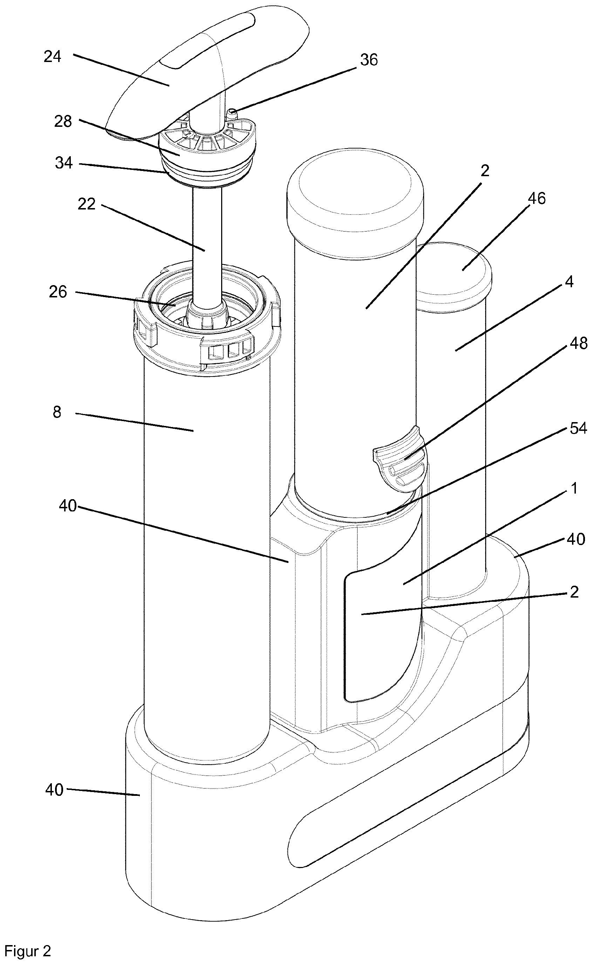

Die Figuren 1 bis 6 zeigen eine erste beispielhafte Ausführungsform einer erfindungsgemäßen Vorrichtung zum Mischen von Polymethylmethacrylat-Knochenzement und zum Lagern der Ausgangskomponenten des Knochenzements, nämlich einer Monomerflüssigkeit und eines Zementpulvers, in verschiedenen Ansichten. Die Vorrichtung ist zum Umsetzen eines erfindungsgemäßen Verfahrens geeignet.

Die Vorrichtung umfasst einen Hohlzylinder 1, in dem ein bereichsweise zylindrischer Pumpkolben 2 axial beweglich angeordnet ist. Eine Öffnungseinrichtung 3 in Form eines kurzen Hebels 3 ist an einer äußeren Seitenwand des Hohlzylinders 1 um eine Achse senkrecht zur axialen Bewegungsrichtung des Pumpkolbens 2 schwenkbar gelagert. Im Zylindermantel des Hohlzylinders 1 befindet sich im Bereich des Hebels 3 eine Aussparung in Form eines Fensters, durch das der Hebel 3 sich in einer Ausgangsstellung (siehe Figuren 1 bis 3) bis ins Innere des Hohlzylinders 1 erstreckt. In den Figuren 1 bis 3 ist dementsprechend die Ausgangsstellung der Vorrichtung gezeigt, die durch die Lage des Hebels 3 und die Position des Pumpkolbens 2 definiert ist.

Direkt neben dem Hebel 3 ist eine Aufnahme 4 für eine Glasampulle 5 vorgesehen, in die die Glasampulle 5 bereits eingesetzt ist. Die Glasampulle 5 ist mit einer Monomerflüssigkeit (nicht dargestellt) gefüllt. Die Glasampulle 5 sitzt an ihrem Boden (in den Figuren 1 bis 3 unten) auf einer Kante beziehungsweise einem Vorsprung auf. Im Bereich des Fensters beziehungsweise der Aussparung im Hohlzylinder 1 ist die Aufnahme 4 nur durch eine verformbare Seitenwand 6 aus Gummi oder einem anderen elastischen Kunststoff begrenzt. An der verformbaren Seitenwand 6 liegt der Hebel 3 mit einer Kante 7 beziehungsweise einem Keil 7 an. Die Glasampulle 5 ist in der Ausgangsstellung (Figuren 1 bis 3) ungeöffnet. Die Ausgangsstellung ist also zum Lagern der Monomerflüssigkeit beziehungsweise der Ausgangskomponenten geeignet.

Auf der anderen Seite neben dem Hohlzylinder 1 mit dem Pumpkolben 2 ist eine Kartusche 8 mit einem zylindrischen Innenraum angeordnet. In der Kartusche 8 befindet sich ein Zementpulver (nicht dargestellt) als zweiter Ausgangskomponente des PMMA-Knochenzements. Die Kartusche 8 ist bodenseitig (in den Figuren 1 bis 6 unten) über eine Verbindungsleitung 9 mit dem Boden des Hohlzylinders 1 verbunden. Die Verbindungsleitung 9 bildet zwischen der Kartusche 8 und dem Hohlzylinder 1 eine Schlaufe 10, die oberhalb des Bodens der Glasampulle 5 angeordnet ist. Hiermit wird verhindert, dass die Monomerflüssigkeit bei geöffneter Glasampulle 5 direkt in den Innenraum der Kartusche 8 fließen kann. Die Verbindungsleitung 9 mündet über einen Stutzen 12 in die Kartusche 8. An dem Stutzen 12 ist ein Außengewinde 14 vorgesehen, auf das ein Innengewinde 16 an der Vorderseite der Kartusche 8 aufgeschraubt ist. Die Kartusche 8 ist also lösbar mit dem Stutzen 12 verbunden. In das Innengewinde 16 kann nach erfolgter Mischung des PMMA-Knochenzements im Innenraum der Kartusche 8 ein Austragsrohr (nicht gezeigt) aufgeschraubt werden, durch das der fertig gemischte PMMA-Knochenzement aus der Kartusche 8 ausgetrieben und appliziert werden kann.

In dem Stutzen 12 befindet sich an der Verbindung zur Kartusche 8 ein pulverundurchlässiger und flüssigkeitsdurchlässiger Filter 18 in der Verbindungsleitung 9. Dieser Filter 18 verhindert, dass das Zementpulver aus dem Innenraum der Kartusche 8 in die Verbindungsleitung 9 rieselt, dort mit der Monomerflüssigkeit reagiert und das aushärtende PMMA die Verbindungsleitung 9 verstopft. In Richtung des Innenraums der Kartusche 8 ist die Einmündung der Verbindungsleitung 9 als Düse ausgebildet. Die Düse ist bevorzugt entsprechend der US 8 662 736 B4 ausgebildet.

Im Innenraum der Kartusche 8 ist eine Mischeinrichtung 20 mit mehreren Mischflügeln 20 vorgesehen, die über einen Mischstab 22 axial im Innenraum der Kartusche 8 beweglich und im Innenraum der Kartusche 8 drehbar ist beziehungsweise sind. Dazu ist der Mischstab 22 durch eine gasdichte Durchführung in den Innenraum der Kartusche 8 geführt. Der Mischstab 22 endet außerhalb der Kartusche 8 in einem Griff 24, mit dem der Mischstab 22 und damit die Mischflügel 20 manuell bewegbar sind. Mit den Mischflügeln 20 kann das Zementpulver mit der Monomerflüssigkeit gemischt werden, nachdem die Monomerflüssigkeit durch die Verbindungsleitung 9 in den Innenraum der Kartusche 8 eingeleitet wurde.

Das hintere Ende der Kartusche 8 (in den Figuren 1 bis 6 oben) ist durch einen zweiteiligen Austragskolben 26, 28 verschließbar. Der Austragskolben 26, 28 besteht aus einem Sterilisationskolben 26 und einem Dichtungskolben 28. Der Sterilisationskolben 26 ist in den Figuren 1 bis 3 bereits in den Innenraum der Kartusche 8 eingesteckt, während der Dichtungskolben 28 in den Figuren 1 bis 3 noch separat zum Sterilisationskolben 26 ist. Wenn der Dichtungskolben 28 noch nicht in den Sterilisationskolben 26 eingesteckt ist, kann die Kartusche 8 einschließlich des Innenraums und des Zementpulvers im Innenraum mit Hilfe eines sterilisierenden Gases wie Ethylenoxid sterilisiert werden. Dazu weist der Sterilisationskolben 26 einen gasdurchlässigen und pulverundurchlässigen Filter 58 auf, der einen Gaseinlass ermöglicht, aber ein Austreten des Zementpulvers aus dem Innenraum der Kartusche 8 verhindert. Anschließend kann der Dichtungskolben 28 in den Sterilisationskolben 26 eingesteckt werden und beide bilden zusammen den Austragskolben 26, 28, mit dem später der im Innenraum der Kartusche 8 fertig gemischte PMMA-Knochenzement durch die vordere Öffnung beziehungsweise ein darin eingeschraubtes Austragsrohr (nicht gezeigt) ausgetrieben werden kann, in dem der Austragskolben 26, 28 in Richtung der Öffnung vorangetrieben wird.

Der Sterilisationskolben 26 weist zwei umlaufende Dichtungen 30 auf, mit denen der Sterilisationskolben 26 gegen die Innenwand der Kartusche 8 abgedichtet ist. Zudem befindet sich an der Vorderseite des Sterilisationskolbens 26 eine Abstreiflippe 32, mit der der gemischte PMMA-Knochenzement von den Innenwänden der Kartusche 8 abgestreift und in der Kartusche 8 vorangetrieben werden kann. Der Dichtungskolben 28 weist ebenfalls eine umlaufende Dichtung 34 auf, mit der er gegen den Sterilisationskolben 26 abgedichtet wird. Am Sterilisationskolben 26 ist eine zusätzliche Dichtung zur weiteren Abdichtung der Verbindung zwischen dem Dichtungskolben 28 und dem Sterilisationskolben 26 vorgesehen. Im Dichtungskolben 28 befindet sich ein Vakuumanschluss 36, der mit einer Durchführung durch den Dichtungskolben 28 verbunden ist. Damit kann der Innenraum der Kartusche 8 auch dann evakuiert werden, wenn der Dichtungskolben 28 in den Sterilisationskolben 26 eingesteckt ist. Hierdurch kann der PMMA-Knochenzement beziehungsweise der Zementteig im Innenraum der Kartusche 8 unter Vakuum beziehungsweise unter Unterdruck gemischt werden, um die Anzahl möglicher Lufteinschlüsse zu reduzieren.

Bodenseitig ist die Vorrichtung durch einen Standfuß 38 mit ebener Auflage begrenzt, auf dem die Vorrichtung auf einer ebenen Unterlage aufgestellt werden kann. Die Inneren Teile der Vorrichtung sind zudem von einem Gehäuse 40 aus Kunststoff umgeben. Der Hohlzylinder 1 ist einteilig mit dem Gehäuse 40 ausgeführt. Nur die Kartusche 8 ist von dem Standfuß 38 und dem Gehäuse 40 zu trennen. Das Gehäuse 40 und der Standfuß 38 können auch gemeinsam als Standfuß 38, 40 aufgefasst werden, da sie zusammen ein zusammenhängendes Teil bilden, das den unteren Teil der Vorrichtung bildet, der die Vorrichtung nach dem Aufstellen hält.

Unterhalb der Glasampulle 5 ist in der Aufnahme 4 ein Filter 42 und/oder ein Sieb 42 angeordnet, mit dem Splitter und Bruchstücke der Glasampulle 5 zurückgehalten werden, wenn diese aufgebrochen wird. Unterhalb des Filters 42 und/oder des Siebs 42 befindet sich eine schräge Ebene, über die die Monomerflüssigkeit aus der geöffneten Glasampulle 5 durch eine Einmündung 44 in den Hohlzylinder 1, also über eine Fluidverbindung 44 zwischen dem Hohlzylinder 1 und der Aufnahme 4 in den Hohlzylinder 1 fließt. Die Oberseite der Aufnahme 4 ist mit einem Deckel 46 verschlossen. In dem Deckel 46 befindet sich ein Rohr 47 aus einem elastischen Kunststoff, beispielsweise einem Schaumstoff oder Gummi, mit dem die Glasampulle 5 auf die Kante am Boden der Aufnahme 4 gedrückt und damit in der Aufnahme 4 positioniert wird.

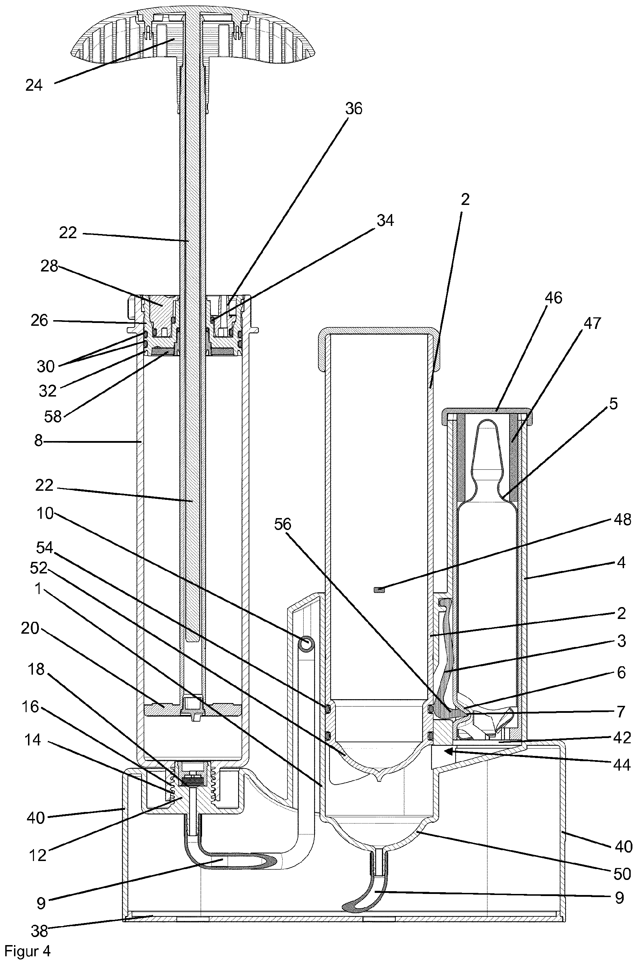

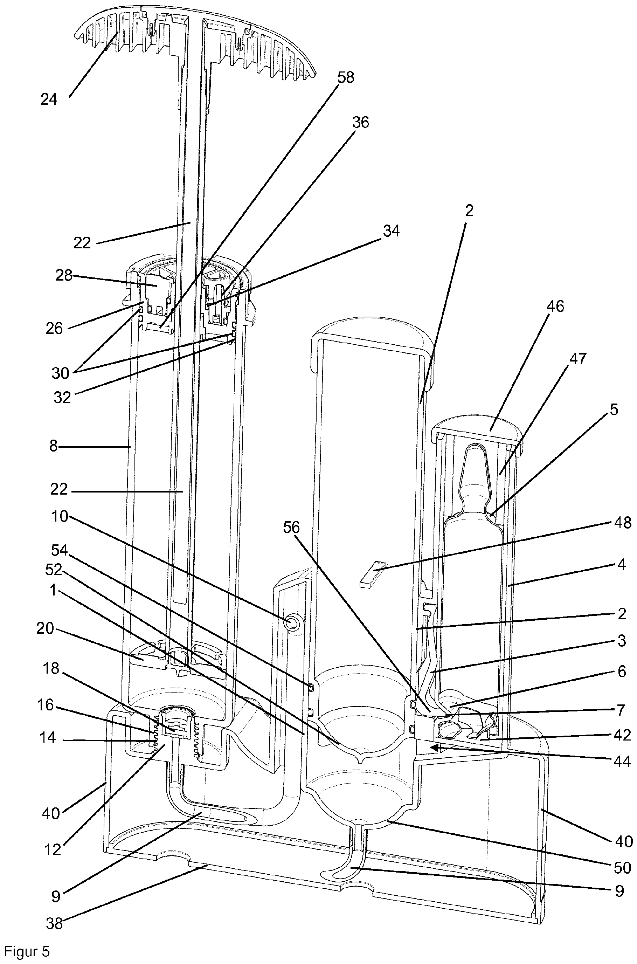

In dem Pumpkolben 2 befindet sich ein Sicherungsstift 48 als Rastvorrichtung 48. An den Sicherungsstift 48 ist ein Griff vorgesehen, mit dem der Sicherungsstift 48 manuell aus dem Pumpkolben 2 gezogen werden kann. Der Sicherungsstift 48 ist durch den Pumpkolben 2 gesteckt und verhindert, dass der Pumpkolben 2 tiefer als bis zum Beginn des Hohlzylinders 1 in den Hohlzylinder 1 eingedrückt werden kann. Wenn der Pumpkolben 2 bis zur Blockade des Sicherungsstifts 48 beziehungsweise der Rastvorrichtung 48 in den Hohlzylinder 1 eingedrückt wurde, wird der Hebel 3 durch die Bewegung des Pumpkolbens 2 um seine Achse geschwenkt und von dem Pumpkolben 2 gegen die verformbare Seitenwand 6 gedrückt. Der Hebel 3 ist dazu so breit, beziehungsweise weist einen derart langen Steg 56 senkrecht zur Schwenkachse auf, dass die Verformung der verformbaren Seitenwand 6 dazu ausreicht, die Glasampulle 5 in der Aufnahme 4 aufzubrechen. Die verformbare Seitenwand 6 verformt sich und die Glasampulle 5 im Inneren der Aufnahme 4 wird aufgebrochen. Der Sicherungsstift 48 verhindert aber noch ein weiteres Eindrücken des Pumpkolbens 2 in den Hohlzylinder 1. Diese Situation ist in den Figuren 4 und 5 dargestellt.

Der Hohlzylinder 1 ist an seiner Grundfläche 50 beziehungsweise an seinem Boden 50 nach Art eines Trichters geformt, an dessen tiefster Stelle sich die Einmündung in die Verbindungsleitung 9 befindet. Der Boden 50 des Hohlzylinders 1 leitet dadurch darin enthaltene Monomerflüssigkeit in die Verbindungsleitung 9. Der Pumpkolben 2 ist an seiner Unterseite 52 passend zum Boden 50 des Hohlzylinders 1 geformt. Dadurch kann mit der unteren Fläche 52 des Pumpkolbens 2 die im Hohlzylinder 1 befindliche Monomerflüssigkeit praktisch vollständig in die Verbindungsleitung 9 gedrückt werden. Zudem ist der höchste Punkt im Hohlzylinder 1 bei der in Figur 4 und 5 gezeigten Situation zudem durch die Einmündung 44 gebildet, so dass Luft aus dem Hohlzylinder 1 durch die Einmündung 44 entweichen kann. Die Monomerflüssigkeit aus der aufgebrochenen Glasampulle 5 fließt also durch den Filter 42 und/oder das Sieb 42 und durch die Einmündung 44 in den Hohlzylinder 1. Die Monomerflüssigkeit wird aber nicht über den durch die Schlaufe 10 gebildeten Scheitelpunkt fließen, wenn sie nicht mit Hilfe des Pumpkolbens 2 darüber hinaus gedrückt wird. Damit wird eine vorzeitige Reaktion der Monomerflüssigkeit mit dem PMMA-Knochenzementpulver in dem Innenraum der Kartusche 8 verhindert.

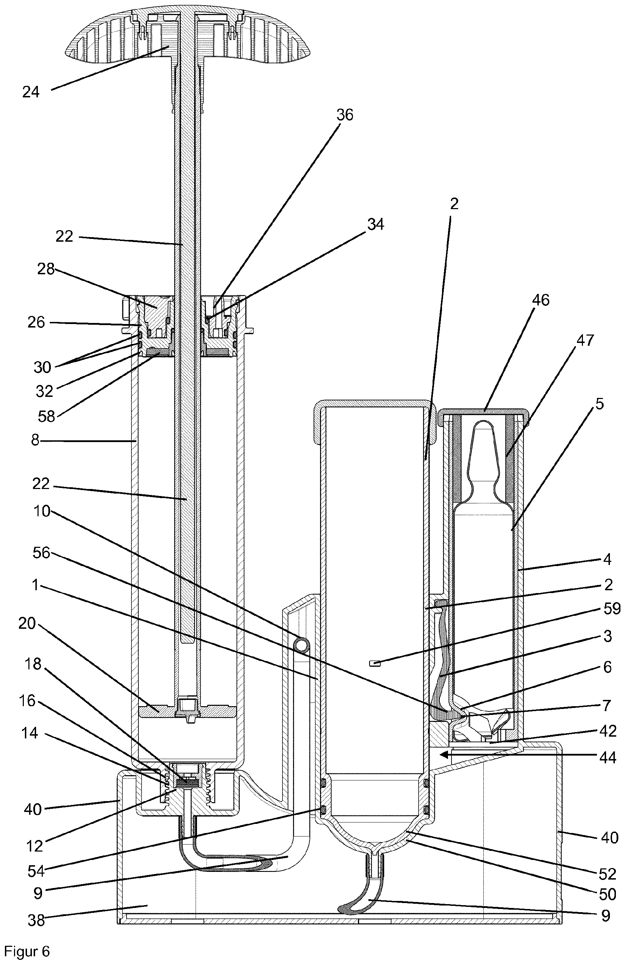

Der Sicherungsstift 48 kann nun aus dem Pumpkolben 2 gezogen werden. Danach kann der Pumpkolben 2 tiefer in den Hohlzylinder 1 eingedrückt werden, um die Monomerflüssigkeit durch die Verbindungsleitung 9 in den Innenraum der Kartusche 8 zu drücken. Die Zeit, die zum Ziehen des Sicherungsstifts 48 benötigt wird, reicht aus, damit die Monomerflüssigkeit von der Aufnahme 4 in den Hohlzylinder 1 fließen kann. Der Pumpkolben 2 ist mit zwei umlaufenden Dichtungen 54 gegen die Innenwand des Hohlzylinders 1 abgedichtet, um zu verhindern, dass sich die Monomerflüssigkeit zwischen dem Pumpkolben 2 und dem Hohlzylinder 1 nach außen drücken kann. Durch vollständiges Einschieben des Pumpkolbens 2 in den Hohlzylinder 1 wird die Monomerflüssigkeit aus dem Hohlzylinder 1 in den Innenraum der Kartusche 8 gedrückt, wo sie mit Hilfe der Mischeinrichtung 20 mit dem Knochenzement gemischt werden kann. Diese Situation ist in Figur 6 gezeigt. Der Sicherungsstift 48 wurde aus dem Pumpkolben 2 gezogen und es verbleibt nur ein Loch 59, in dem zuvor der Sicherungsstift 48 gesteckt hatte. Beim Mischen kann über den Vakuumanschluss 36 im Innenraum der Kartusche 8 ein Vakuum beziehungsweise ein Unterdruck erzeugt werden.

Der im Innenraum der Kartusche 8 gemischte PMMA-Knochenzement kann angewendet werden, indem die Kartusche 8 von der restlichen Vorrichtung abgeschraubt wird, ein Austragsrohr in das Innengewinde 16 geschraubt wird und die Kartusche 8 in eine Austragseinrichtung in Form einer Auspresspistole eingesetzt wird, mit der der Austragskolben 26, 28 in Richtung der vorderen Öffnung nach vorne getrieben wird und dabei der fertig gemischte Knochenzement aus dem Innenraum der Kartusche 8 ausgepresst wird. Der Pumpkolben 2 wird bei der ersten Ausführungsform nach den Figuren 1 bis 6 manuell angetrieben, das heißt, mit der Hand in den Hohlzylinder 1 eingedrückt.

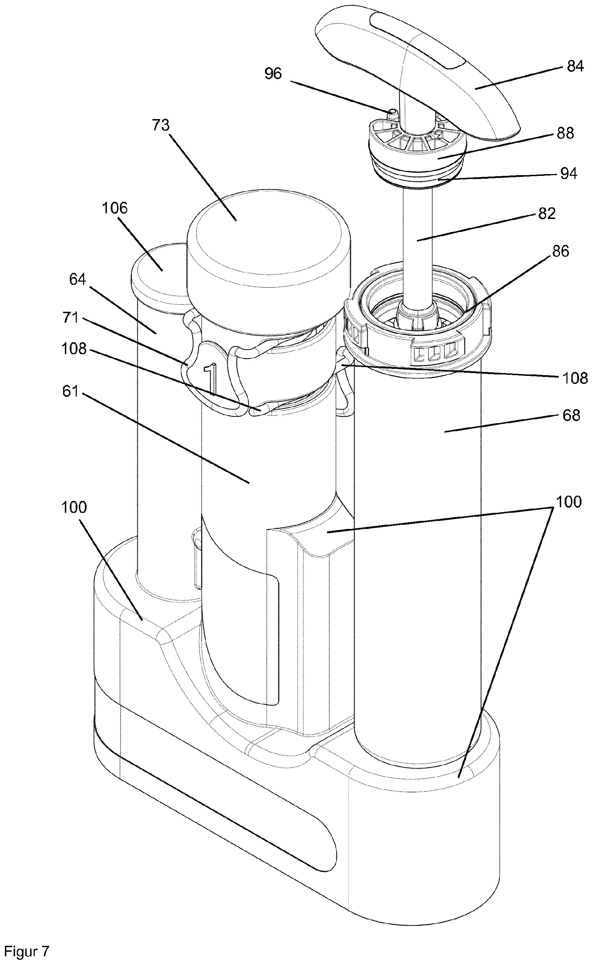

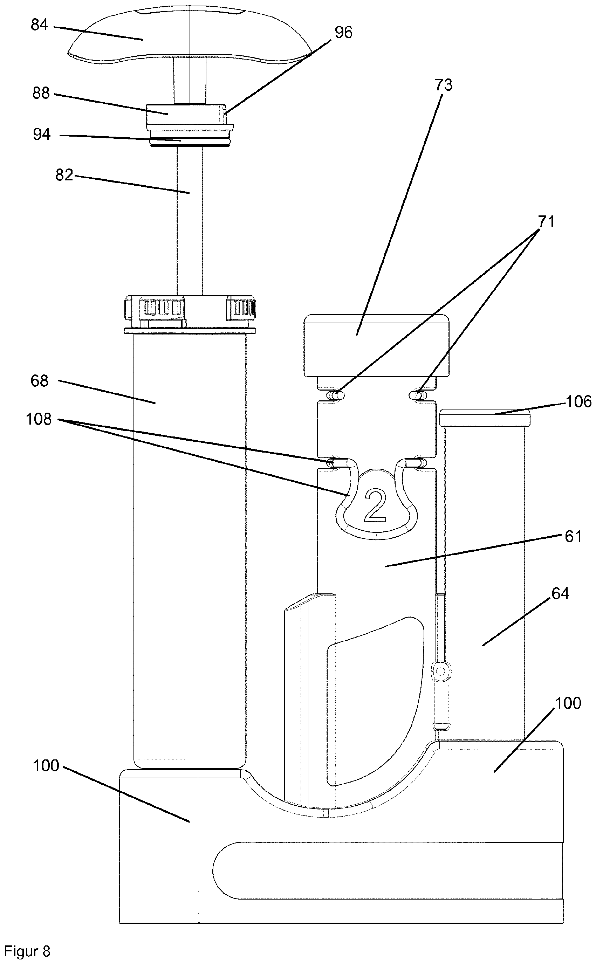

Die Figuren 7 bis 12 zeigen Darstellungen einer zweiten alternativen beispielhaften Ausführungsform einer erfindungsgemäßen Vorrichtung zum Mischen von Polymethylmethacrylat-Knochenzement und zum Lagern der Ausgangskomponenten des Knochenzements, nämlich einer Monomerflüssigkeit und eines Zementpulvers, bei der ein Pumpkolben 62 nicht manuell, sondern mittels eines internen Energiespeichers 75 angetrieben und in einen Hohlzylinder 61 eingedrückt wird. Auch diese Vorrichtung ist zum Umsetzen eines erfindungsgemäßen Verfahrens geeignet.

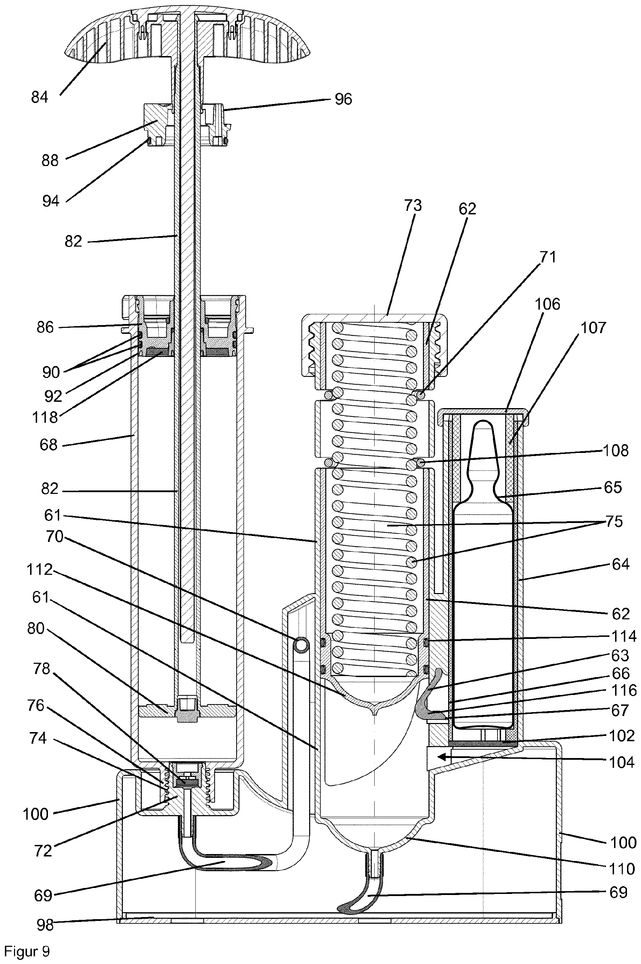

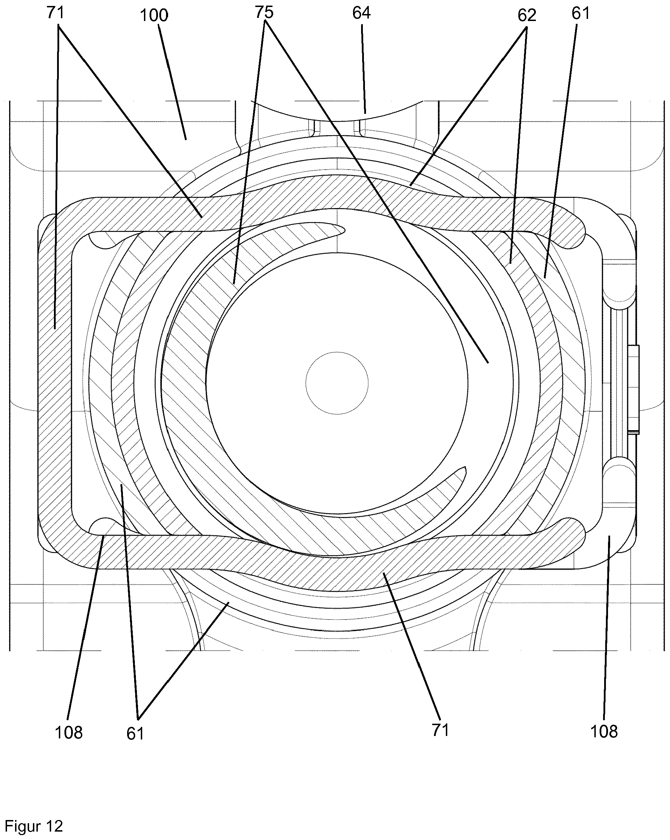

Die zweite alternative Vorrichtung umfasst einen Hohlzylinder 61, in dem ein bereichsweise zylindrischer Pumpkolben 62 axial beweglich angeordnet ist. Die beiden in den Querschnittansichten der Figuren 9, 10 und 11 zu erkennenden Teilbereiche des Pumpkolbens 62 sind fest miteinander verbunden. Der Pumpkolben 62 ist also einteilig ausgeführt, wie auch in Figur 12 zu erkennen ist. Eine Öffnungseinrichtung 63 in Form eines Hebels 63 ist an einer äußeren Seitenwand des Hohlzylinders 61 um eine Achse senkrecht zur axialen Bewegungsrichtung des Pumpkolbens 62 schwenkbar gelagert. Im Zylindermantel des Hohlzylinders 61 befindet sich im Bereich des Hebels 63 eine Aussparung in Form eines Fensters, durch das der Hebel 63 sich in einer Ausgangsstellung (siehe Figur 9) bis ins Innere des Hohlzylinders 61 erstreckt. In den Figuren 7 bis 9 und 12 ist die Ausgangsstellung der zweiten alternativen Vorrichtung gezeigt, die durch die Lage des Hebels 63 und die Position des Pumpkolbens 62 definiert ist.

Direkt neben dem Hebel 63 ist eine Aufnahme 64 für eine Glasampulle 65 vorgesehen, in die die Glasampulle 65 bereits eingesetzt ist. Die Glasampulle 65 ist mit einer Monomerflüssigkeit (nicht dargestellt) gefüllt. Die Glasampulle 65 sitzt an ihrem Boden (in den Figuren 7 bis 9 unten) auf einer Kante beziehungsweise einem Vorsprung auf. Im Bereich des Fensters beziehungsweise der Aussparung im Hohlzylinder 61 ist die Aufnahme 64 nur durch eine verformbare Seitenwand 66 aus Gummi oder einem anderen elastischen Kunststoff begrenzt. An der verformbaren Seitenwand 66 liegt der Hebel 63 mit einer Kante 67 beziehungsweise einem Keil 67 an. Die Glasampulle 65 ist in der Ausgangsstellung (Figuren 7 bis 9 und 12) ungeöffnet. Die Ausgangsstellung ist also zum Lagern der Monomerflüssigkeit beziehungsweise der Ausgangskomponenten geeignet.

Auf der anderen Seite neben dem Hohlzylinder 61 mit dem Pumpkolben 62 ist eine Kartusche 68 mit einem zylindrischen Innenraum angeordnet. In der Kartusche 68 befindet sich ein Zementpulver (nicht dargestellt) als zweiter Ausgangskomponente des PMMA-Knochenzements. Die Kartusche 68 ist bodenseitig (in den Figuren 7 bis 12 unten) über eine Verbindungsleitung 69 mit dem Boden des Hohlzylinders 61 verbunden. Die Verbindungsleitung 69 bildet zwischen der Kartusche 68 und dem Hohlzylinder 61 eine Schlaufe 70, die oberhalb des Bodens der Glasampulle 65 angeordnet ist. Hiermit wird verhindert, dass die Monomerflüssigkeit bei geöffneter Glasampulle 65 direkt in den Innenraum der Kartusche 68 fließen kann. Der Pumpkolben 62 kann in der Ausgangsstellung (Figuren 7 bis 9 und 12) nicht in den Hohlzylinder 61 eingedrückt werden, da der Pumpkolben 62 mit Hilfe einer Spange 71 als Rastmittel 71 mit dem Hohlzylinder 61 verbunden ist. Erst, nachdem die Spange 71 entfernt wurde, kann der Pumpkolben 62 in den Hohlzylinder 61 gedrückt werden.

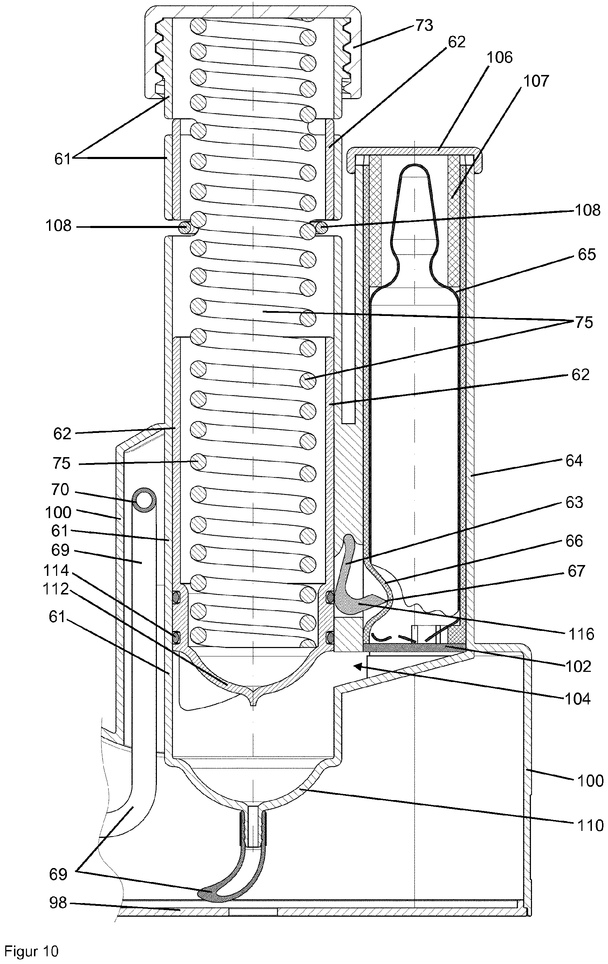

Die Verbindungsleitung 69 mündet über einen Stutzen 72 in die Kartusche 68. Die Oberseite des Hohlzylinders 61 ist mit einer aufgeschraubten Kappe 73 verschlossen, gegen die sich eine gespannte Druckfeder 75 zum Antreiben des Pumpkolbens 62 abstützt. Die gespannte Druckfeder 75 stützt sich zu diesem Zweck gegen die Kappe 73 und den Pumpkolben 62 ab und steht zumindest in der in den Figuren 7 bis 9 und 12 gezeigten Ausgangsstellung und auch in der in Figur 10 gezeigten Zwischenstellung unter Druckspannung, so dass der Pumpkolben 62 mit der Druckfeder 75 bis zum Anschlag in dem Hohlzylinder 61 bewegt werden kann. Bevorzugt ist die Druckfeder 75 auch dann noch gespannt. Die genaue Anordnung der ersten Spange 71 ist in Figur 12 als eine schematische Querschnittansicht eines Teilbereichs der zweiten erfindungsgemäßen Vorrichtung gezeigt, wobei die Schnittebene parallel zur ersten Spange 71 beziehungsweise dem Rastmittel 71 verläuft. Die Druckfeder 75 besteht bevorzugt aus einem Federstahl.

An dem Stutzen 72 ist ein Außengewinde 74 vorgesehen, auf das ein Innengewinde 76 an der Vorderseite der Kartusche 68 aufgeschraubt ist. Die Kartusche 68 ist also lösbar mit dem Stutzen 72 verbunden. In das Innengewinde 76 kann nach erfolgter Mischung des PMMA-Knochenzements im Innenraum der Kartusche 68 ein Austragsrohr (nicht gezeigt) aufgeschraubt werden, durch das der fertig gemischte PMMA-Knochenzement aus der Kartusche 68 ausgetrieben und appliziert werden kann.

In dem Stutzen 72 befindet sich an der Verbindung zur Kartusche 68 ein pulverundurchlässiger und flüssigkeitsdurchlässiger Filter 78 in der Verbindungsleitung 69. Dieser Filter 78 verhindert, dass das Zementpulver aus dem Innenraum der Kartusche 68 in die Verbindungsleitung 69 rieselt, dort mit der Monomerflüssigkeit reagiert und das aushärtende PMMA die Verbindungsleitung 69 verstopft. In Richtung des Innenraums der Kartusche 68 ist die Einmündung der Verbindungsleitung 69 als Düse ausgebildet. Die Düse ist bevorzugt entsprechend der US 8 662 736 B4 ausgebildet.

Im Innenraum der Kartusche 68 ist eine Mischeinrichtung 80 mit mehreren Mischflügeln 80 vorgesehen, die über einen Mischstab 82 axial im Innenraum der Kartusche 68 beweglich und im Innenraum der Kartusche 68 drehbar ist beziehungsweise sind. Dazu ist der Mischstab 82 durch eine gasdichte Durchführung in den Innenraum der Kartusche 68 geführt. Der Mischstab 82 endet außerhalb der Kartusche 68 in einem Griff 84, mit dem der Mischstab 82 und damit die Mischflügel 80 manuell bewegbar sind. Mit den Mischflügeln 80 kann das Zementpulver mit der Monomerflüssigkeit gemischt werden, nachdem die Monomerflüssigkeit durch die Verbindungsleitung 69 in den Innenraum der Kartusche 68 eingeleitet wurde.

Das hintere Ende der Kartusche 68 (in den Figuren 7 bis 12 oben) ist durch einen zweiteiligen Austragskolben 86, 88 verschließbar. Der Austragskolben 86, 88 besteht aus einem Sterilisationskolben 86 und einem Dichtungskolben 88. Der Sterilisationskolben 86 ist in den Figuren 7 bis 9 bereits in den Innenraum der Kartusche 68 eingesteckt, während der Dichtungskolben 88 in den Figuren 7 bis 9 noch separat zum Sterilisationskolben 86 ist. Wenn der Dichtungskolben 88 noch nicht in den Sterilisationskolben 86 eingesteckt ist, kann die Kartusche 68 einschließlich des Innenraums und des Zementpulvers im Innenraum mit Hilfe eines sterilisierenden Gases wie Ethylenoxid sterilisiert werden. Dazu weist der Sterilisationskolben 86 einen gasdurchlässigen und pulverundurchlässigen Filter 118 auf, der einen Gaseinlass ermöglicht, aber ein Austreten des Zementpulvers aus dem Innenraum der Kartusche 68 verhindert. Anschließend kann der Dichtungskolben 88 in den Sterilisationskolben 86 eingesteckt werden und beide bilden zusammen den Austragskolben 86, 88, mit dem später der im Innenraum der Kartusche 68 fertig gemischte PMMA-Knochenzement durch die vordere Öffnung beziehungsweise ein darin eingeschraubtes Austragsrohr (nicht gezeigt) ausgetrieben werden kann, in dem der Austragskolben 86, 88 in Richtung der Öffnung vorangetrieben wird.

Der Sterilisationskolben 86 weist zwei umlaufende Dichtungen 90 auf, mit denen der Sterilisationskolben 86 gegen die Innenwand der Kartusche 68 abgedichtet ist. Zudem befindet sich an der Vorderseite des Sterilisationskolbens 86 eine Abstreiflippe 92, mit der der gemischte PMMA-Knochenzement von den Innenwänden der Kartusche 68 abgestreift und in der Kartusche 68 vorangetrieben werden kann. Der Dichtungskolben 88 weist ebenfalls eine umlaufende Dichtung 94 auf, mit der er gegen den Sterilisationskolben 86 abgedichtet wird. Am Sterilisationskolben 86 ist eine zusätzliche Dichtung zur weiteren Abdichtung der Verbindung zwischen dem Dichtungskolben 88 und dem Sterilisationskolben 86 vorgesehen. Im Dichtungskolben 88 befindet sich ein Vakuumanschluss 96, der mit einer Durchführung durch den Dichtungskolben 88 verbunden ist. Damit kann der Innenraum der Kartusche 68 auch dann evakuiert werden, wenn der Dichtungskolben 88 in den Sterilisationskolben 86 eingesteckt ist. Hierdurch kann der PMMA-Knochenzement beziehungsweise der Zementteig im Innenraum der Kartusche 68 unter Vakuum beziehungsweise unter Unterdruck gemischt werden, um die Anzahl möglicher Lufteinschlüsse zu reduzieren.

Bodenseitig ist die Vorrichtung durch einen Standfuß 98 mit ebener Auflage begrenzt, auf dem die Vorrichtung auf einer ebenen Unterlage aufgestellt werden kann. Die Inneren Teile der Vorrichtung sind zudem von einem Gehäuse 100 aus Kunststoff umgeben. Der Hohlzylinder 61 ist einteilig mit dem Gehäuse 100 ausgeführt. Nur die Kartusche 68 ist von dem Standfuß 98 und dem Gehäuse 100 zu trennen. Das Gehäuse 100 und der Standfuß 98 können auch gemeinsam als Standfuß 98, 100 aufgefasst werden, da sie zusammen ein zusammenhängendes Teil bilden, das den unteren Teil der Vorrichtung bildet, der die Vorrichtung nach dem Aufstellen hält.

Unterhalb der Glasampulle 65 ist in der Aufnahme 64 ein Filter 102 und/oder ein Sieb 102 angeordnet, mit dem Splitter und Bruchstücke der Glasampulle 65 zurückgehalten werden, wenn diese aufgebrochen wird. Unterhalb des Filters 102 und/oder des Siebs 102 befindet sich eine schräge Ebene, über die die Monomerflüssigkeit aus der geöffneten Glasampulle 65 durch eine Einmündung 104 in den Hohlzylinder 61, also über eine Fluidverbindung 104 zwischen dem Hohlzylinder 61 und der Aufnahme 64 in den Hohlzylinder 61 fließt. Die Oberseite der Aufnahme 64 ist mit einem Deckel 106 verschlossen. In dem Deckel 106 befindet sich ein Rohr 107 aus einem elastischen Kunststoff, beispielsweise einem Schaumstoff oder Gummi, mit dem die Glasampulle 65 auf die Kante am Boden der Aufnahme 64 gedrückt und damit in der Aufnahme 64 positioniert wird.

Eine zweite Spange 108 ist als Rastvorrichtung 108 in passende Ausnehmungen in dem Hohlzylinder 61 und dem Pumpkolben 62 vorgesehen. Die zweite Spange 108 verhindert, dass der Pumpkolben 62 tiefer als bis zur Einmündung 104 in den Hohlzylinder 61 eingedrückt werden kann. Nachdem also die erste Spange 71 entfernt wurde, drückt die Druckfeder 75 den Pumpkolben 62 in den Hohlzylinder 61, bis der Pumpkolben 62 von der zweiten Spange 108 in der Zwischenposition gehalten beziehungsweise aufgehalten wird. Diese Position ist in Figur 10 gezeigt.

An den beiden Spangen 71, 108 sind Griffe vorgesehen, mit denen sie manuell aus dem aus den Ausnehmungen gezogen werden können. Zudem sind auf den Griffen der Spangen 71, 108 die Zahlen "1" und "2" als Beschriftungen vorgesehen, um dem Anwender die Reihenfolge vorzugeben, in der die beiden Spangen 71, 108 herausgezogen werden sollen. Wenn der Pumpkolben 62 von der Druckfeder 75 bis zur Blockade durch die zweite Spange 108 beziehungsweise der Rastvorrichtung 108 in den Hohlzylinder 61 eingedrückt wurde, wird der Hebel 63 durch die Bewegung des Pumpkolbens 62 um seine Achse geschwenkt und von dem Pumpkolben 62 gegen die verformbare Seitenwand 66 gedrückt. Der Hebel 63 ist dazu so breit, beziehungsweise weist einen derart langen Steg 116 senkrecht zur Schwenkachse auf, dass die Verformung der verformbaren Seitenwand 66 dazu ausreicht, die Glasampulle 65 in der Aufnahme 64 aufzubrechen. Die verformbare Seitenwand 66 verformt sich und die Glasampulle 65 im Inneren der Aufnahme 64 wird aufgebrochen. Die zweite Spange 108 verhindert aber noch ein weiteres Eindrücken des Pumpkolbens 62 in den Hohlzylinder 61. Diese Situation ist in Figur 10 dargestellt.

Der Hohlzylinder 61 ist an seiner Grundfläche 110 beziehungsweise an seinem Boden 110 nach Art eines Trichters geformt, an dessen tiefster Stelle sich die Einmündung in die Verbindungsleitung 69 befindet. Der Boden 110 des Hohlzylinders 61 leitet dadurch darin enthaltene Monomerflüssigkeit in die Verbindungsleitung 69. Der Pumpkolben 62 ist an seiner Unterseite 112 passend zum Boden 110 des Hohlzylinders 61 geformt. Dadurch kann mit der unteren Fläche 112 des Pumpkolbens 62 die im Hohlzylinder 61 befindliche Monomerflüssigkeit praktisch vollständig in die Verbindungsleitung 69 gedrückt werden. Zudem ist der höchste Punkt im Hohlzylinder 61 bei der in Figur 10 gezeigten Situation zudem durch die Einmündung 104 gebildet, so dass Luft aus dem Hohlzylinder 61 durch die Einmündung 104 entweichen kann. Die Monomerflüssigkeit aus der aufgebrochenen Glasampulle 65 fließt also durch den Filter 102 und/oder das Sieb 102 und durch die Einmündung 104 in den Hohlzylinder 61. Die Monomerflüssigkeit wird aber nicht über den durch die Schlaufe 70 gebildeten Scheitelpunkt fließen, wenn sie nicht mit Hilfe des Pumpkolbens 62 darüber hinaus gedrückt wird. Damit wird eine vorzeitige Reaktion der Monomerflüssigkeit mit dem PMMA-Knochenzementpulver in dem Innenraum der Kartusche 68 verhindert.

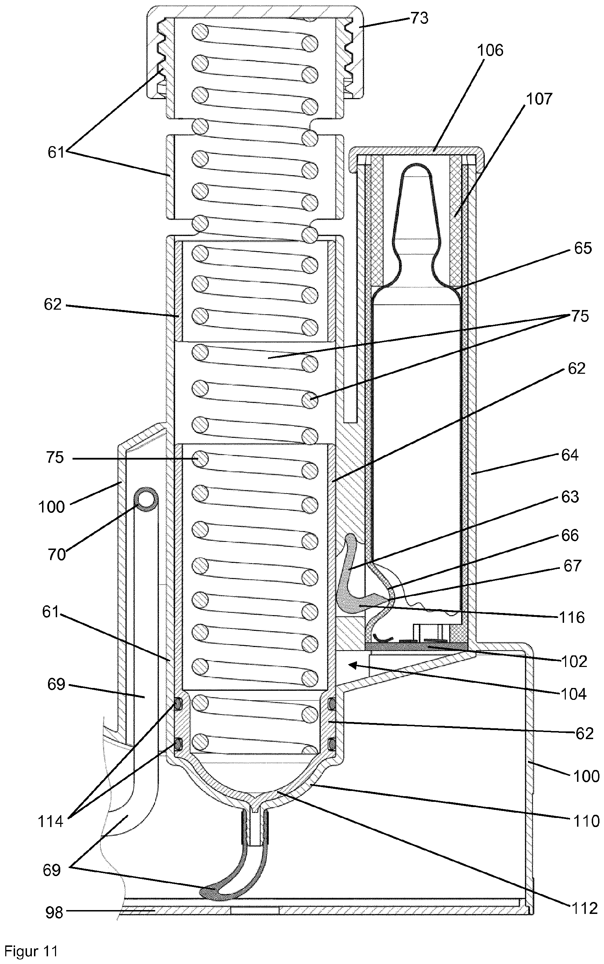

Die zweite Spange 108 kann nun aus dem Pumpkolben 62 gezogen werden. Danach wird der Pumpkolben 62 von der Druckfeder 75 tiefer in den Hohlzylinder 61 eingedrückt und dabei die Monomerflüssigkeit durch die Verbindungsleitung 69 in den Innenraum der Kartusche 68 gedrückt. Die Zeit, die zum Ziehen der zweiten Spange 108 benötigt wird, reicht aus, damit die Monomerflüssigkeit von der Aufnahme 64 in den Hohlzylinder 61 fließen kann. Der Pumpkolben 62 ist mit zwei umlaufenden Dichtungen 114 gegen die Innenwand des Hohlzylinders 61 abgedichtet, um zu verhindern, dass sich die Monomerflüssigkeit zwischen dem Pumpkolben 62 und dem Hohlzylinder 61 nach außen drücken kann. Durch vollständiges Einschieben des Pumpkolbens 62 in den Hohlzylinder 61 wird die Monomerflüssigkeit aus dem Hohlzylinder 61 in den Innenraum der Kartusche 68 gedrückt, wo sie mit Hilfe der Mischeinrichtung 80 mit dem Knochenzement gemischt werden kann. Diese Situation ist in Figur 11 dargestellt. Die zweite Spange 108 wurde herausgezogen. Beim Mischen kann über den Vakuumanschluss 96 im Innenraum der Kartusche 68 ein Vakuum beziehungsweise ein Unterdruck erzeugt werden.

Der im Innenraum der Kartusche 68 gemischte PMMA-Knochenzement kann angewendet werden, indem die Kartusche 68 von der restlichen Vorrichtung abgeschraubt wird, ein Austragsrohr in das Innengewinde 76 geschraubt wird und die Kartusche 68 in eine Austragseinrichtung in Form einer Auspresspistole eingesetzt wird, mit der der Austragskolben 86, 88 in Richtung der vorderen Öffnung nach vorne getrieben wird und dabei der fertig gemischte Knochenzement aus dem Innenraum der Kartusche 68 ausgepresst wird. Der Pumpkolben 62 wird bei der zweiten Ausführungsform nach den Figuren 7 bis 12 im Gegensatz zur ersten Ausführungsform also nicht manuell, sondern mit einer gespannten Druckfeder 75 angetrieben, das heißt, mit der Druckfeder 75 in den Hohlzylinder 61 eingedrückt.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 61: Hohlzylinder
- 2, 62: Pumpkolben
- 3, 63: Öffnungseinrichtung / Hebel
- 4, 64: Aufnahme
- 5, 65: Glasampulle
- 6, 66: Verformbare Seitenwand
- 7, 67: Keil / Kante
- 8, 68: Kartusche
- 9, 69: Verbindungsleitung
- 10, 70: Schlaufe
- 12, 72: Stutzen
- 14, 74: Außengewinde
- 16, 76: Innengewinde
- 18, 78: Pulverundurchlässiger und Flüssigkeitsdurchlässiger Filter
- 20, 80: Mischflügel
- 22, 82: Mischstab
- 24, 84: Griff
- 26, 86: Sterilisationskolben
- 28, 88: Dichtungskolben
- 30, 90: Dichtung
- 32, 92: Abstreiflippe
- 34, 94: Dichtung
- 36, 96: Vakuumanschluss
- 38, 98: Standfuß
- 40, 100: Gehäuse
- 42, 102: Sieb / Filter
- 44, 104: Einmündung in den Hohlzylinder / Fluidverbindung
- 46, 106: Deckel
- 47, 107: Rohr
- 48: Sicherungsstift mit Griff / Rastvorrichtung
- 50, 110: Boden des Hohlzylinders
- 52, 112: Untere Fläche des Pumpkolbens
- 54, 114: Dichtung
- 56, 116: Steg / Querstück des Hebels
- 58, 118: Gasdurchlässiger und Pulverundurchlässiger Filter
- 59: Durchführung
- 71: Spange / Rastmittel
- 73: Schraubkappe
- 75: Druckfeder
- 108: Spange / Rastvorrichtung

## Patentansprüche

1. Vorrichtung zum Mischen von Polymethylmethacrylat-Knochenzement und zum Lagern der Ausgangskomponenten des Knochenzements, insbesondere einer Monomerflüssigkeit und eines Zementpulvers als Ausgangskomponenten des Knochenzements, die Vorrichtung aufweisend
eine Kartusche (8, 68) mit einem Innenraum zum Mischen des Knochenzements, wobei der Innenraum auf einer Seite durch einen beweglichen Austragskolben (26, 28, 86, 88) verschlossen ist,
eine Aufnahme (4, 64) mit zumindest bereichsweise geschlossenen Seitenwänden zur Aufnahme eines Monomerflüssigkeitsbehälters (5, 65), wobei die Aufnahme (4, 64) zumindest eine verformbare geschlossene Seitenwand (6, 66) aufweist,
ein Sieb (42, 102) und/oder ein Filter (42, 102) das, der oder die unterhalb der Aufnahme (4, 64) angeordnet ist oder sind, so dass der Inhalt des geöffneten Monomerflüssigkeitsbehälters (5, 65) durch das Sieb (42, 102) und/oder den Filter (42, 102) fließt,
eine Verbindungsleitung (9, 69), durch die die Monomerflüssigkeit in den Innenraum der Kartusche (8, 68) zu leiten ist,
einen Hohlzylinder (1, 61), der mit der Verbindungsleitung (9, 69) verbunden ist und der über eine Fluidverbindung (44, 104) mit der Aufnahme (4, 64) verbunden ist, so dass der Hohlzylinder (1, 61) in einer Fluidleitung (9, 44, 69, 104) zwischen der Aufnahme (4, 64) und dem Innenraum der Kartusche (8, 68) angeordnet ist, wobei in dem Hohlzylinder (1, 61) ein axial im Hohlzylinder (1, 61) verschiebbarer Pumpkolben (2, 62) angeordnet ist, und
eine Öffnungseinrichtung (3, 63), die beweglich gegen die verformbare Seitenwand (6, 66) der Aufnahme (4, 64) gelagert ist, wobei die Öffnungseinrichtung (3, 63) sich bis in den Hohlzylinder (1, 61) erstreckt, wobei die Öffnungseinrichtung (3, 63) durch Eindrücken des Pumpkolbens (2, 62) in den Hohlzylinder (1, 61) gegen die verformbare Seitenwand (6, 66) der Aufnahme (4, 64) zu drücken ist, so dass sich die verformbare Seitenwand (6, 66) derart verformt, dass ein in der Aufnahme (4, 64) befindlicher Monomerflüssigkeitsbehälter (5, 65) durch den Druck der Öffnungseinrichtung (3, 63) zu öffnen ist.

2. Vorrichtung nach Anspruch 1, **dadurch gekennzeichnet, dass** die Monomerflüssigkeit aus dem geöffneten Monomerflüssigkeitsbehälter (5, 65) in den Hohlzylinder (1, 61) fließen kann und die Verbindungsleitung (9, 69) den Hohlzylinder (1, 61) mit dem Innenraum der Kartusche (8, 68) derart verbindet, dass mit dem Pumpkolben (2, 62) Monomerflüssigkeit aus dem Hohlzylinder (1, 61) durch Eindrücken des Pumpkolbens (2, 62) in den Hohlzylinder (1, 61) durch die Verbindungsleitung (9, 69) in den Innenraum der Kartusche (8, 68) zu drücken ist.

3. Vorrichtung nach einem der vorangehenden Ansprüche, **dadurch gekennzeichnet, dass**
zwischen der Verbindungsleitung (9, 69) und dem Innenraum der Kartusche (8, 68) ein für das Zementpulver undurchlässiger und für die Monomerflüssigkeit durchlässiger Filter (18, 78) angeordnet ist.

4. Vorrichtung nach einem der vorangehenden Ansprüche, **dadurch gekennzeichnet, dass**
die Vorrichtung einen Standfuß (38, 40, 98, 100) aufweist, in dem zumindest ein Teil der Verbindungsleitung (9, 69) angeordnet ist, wobei die Kartusche (8, 68) lösbar mit dem Standfuß (38, 40, 98, 100) verbunden ist, insbesondere über ein Schraubgewinde (14, 16, 74, 76) lösbar mit dem Standfuß (38, 40, 98, 100) verbunden ist, wobei bevorzugt gegebenenfalls der für das Zementpulver undurchlässige und für die Monomerflüssigkeit durchlässige Filter (18, 78) in dem Standfuß (38, 40, 98, 100) der Vorrichtung angeordnet ist, besonders bevorzugt in der Verbindung (12, 72) zur Kartusche (8, 68) des Standfußes (38, 40, 98, 100) angeordnet ist.

5. Vorrichtung nach einem der vorangehenden Ansprüche, **dadurch gekennzeichnet, dass**
die Aufnahme (4, 64) an einer Mantelfläche des Hohlzylinders (1, 61) in den Hohlzylinder (1, 61) mündet.

6. Vorrichtung nach einem der vorangehenden Ansprüche, **dadurch gekennzeichnet, dass**
der Hohlzylinder (1, 61) auf der dem Pumpkolben (2, 62) gegenüberliegenden Seite einen kegelförmigen, halbkugelförmigen oder anderen sich nach unten verjüngenden Boden (50, 110) aufweist, wobei bevorzugt die dem Boden (50, 110) des Hohlzylinders (1, 61) zugewandte Fläche (52, 112) des Pumpkolbens (2, 62) eine Negativform des Bodens (50, 110) bildet.

7. Vorrichtung nach einem der vorangehenden Ansprüche, **dadurch gekennzeichnet, dass**
die Vorrichtung eine gespannte Druckfeder (75) und zumindest eine Arretierung (48, 71, 108) aufweist, wobei die Druckfeder (75) und/oder der Pumpkolben (2, 62) mit der Arretierung (48, 71, 108) lösbar arretiert ist oder sind, wobei bei gelöster Arretierung (48, 71, 108) die Druckfeder (75) einen Druck auf den Pumpkolben (2, 62) ausübt, so dass der Pumpkolben (2, 62) in den Hohlzylinder (1, 61) gepresst wird.

8. Vorrichtung nach einem der vorangehenden Ansprüche, **dadurch gekennzeichnet, dass**
die Verbindungsleitung (9, 69) zwischen dem Hohlzylinder (1, 61) und dem Innenraum der Kartusche (8, 68) eine nach oben weisende Schlaufe (10, 70) aufweist, wobei der höchste Punkt der Schlaufe (10, 70) oberhalb der Einmündung (44, 104) der Aufnahme (4, 64) in den Hohlzylinder (1, 61) liegt.

9. Vorrichtung nach einem der vorangehenden Ansprüche, **dadurch gekennzeichnet, dass**
das Volumen im Hohlzylinder (1, 61) kleiner oder gleich dem Volumen der Monomerflüssigkeit in dem Monomerflüssigkeitsbehälter (5, 65) ist.

10. Vorrichtung nach einem der vorangehenden Ansprüche, **dadurch gekennzeichnet, dass**
die Öffnungseinrichtung (3, 63) ein Hebel (3, 63) ist, der um eine Achse drehbar gegen die Aufnahme (4, 64) gelagert ist, wobei ein freies Ende (7, 67) des Hebels (3, 63) von dem Pumpkolben (2, 62) gegen die verformbare Seitenwand (6, 66) der Aufnahme (4, 64) drückbar ist, so dass das freie Ende (7, 67) des Hebels (3, 63) die verformbare Seitenwand (6, 66) derart verformt, dass ein in der Aufnahme (4, 64) befindlicher und zur Aufnahme (4, 64) passender Monomerflüssigkeitsbehälter (5, 65) durch den Druck des freien Endes (7, 67) des Hebels (3, 63) zu öffnen ist, insbesondere aufzubrechen oder aufzuschlitzen oder aufzustechen ist.

11. Vorrichtung nach einem der vorangehenden Ansprüche, **dadurch gekennzeichnet, dass**
die Aufnahme (4, 64) ein Hohlzylinder (1, 61) ist und/oder die Aufnahme (4, 64) aus einem Elastomer besteht oder einen Einsatz aus einem Elastomer umfasst, wobei vorzugsweise das Elastomer eine Shore-Härte größer 60 aufweist, wobei besonders bevorzugt das Elastomer ein Silikonkautschuk oder ein Ethylen-Propylen-Dien-Kautschuk (EPDM) ist.

12. Vorrichtung nach einem der vorangehenden Ansprüche, **dadurch gekennzeichnet, dass**
in der Aufnahme (4, 64) ein Absatz zur Auflage des Monomerflüssigkeitsbehälters (5, 65) angeordnet ist, wobei der Absatz kleiner als die Hälfte der Fläche des Bodens des Monomerflüssigkeitsbehälters (5, 65) oder des Querschnitts des Monomerflüssigkeitsbehälters (5, 65) ist.

13. Vorrichtung nach einem der vorangehenden Ansprüche, **dadurch gekennzeichnet, dass**
ein freies Ende (7, 67) der Öffnungseinrichtung (3, 63) bei einer Bewegung des Pumpkolbens (2, 62) in den Hohlzylinder (1, 61) hinein derart auf die verformbare Seitenwand (6, 66) drückt, dass der Vektor der Kraft eine Komponente aufweist, die in Richtung des Siebs (42, 102) und/oder Filters (42, 102) gerichtet ist und/oder die einen in die Aufnahme (4, 64) eingesetzter Monomerflüssigkeitsbehälter (5, 65) in die Aufnahme (4, 64) hineindrückt, vorzugsweise in Richtung des Absatzes drückt.

14. Vorrichtung nach einem der vorangehenden Ansprüche, **dadurch gekennzeichnet, dass**
der Pumpkolben (2, 62) mit einer manuell lösbaren Rastvorrichtung (48, 108) fixierbar ist, die nach axialer Verschiebung des Pumpkolbens (2, 62) über die Öffnungseinrichtung (3, 63) im Hohlzylinder (1, 61), den Pumpkolben (2, 62) oberhalb der Einmündung der Fluidverbindung (44, 104) zur Aufnahme (4, 64) im Hohlzylinder (1, 61) blockiert, so dass der Pumpkolben (2, 62) im Hohlzylinder (1, 61) nicht über die Einmündung der Fluidverbindung (44, 104) bewegbar ist, wenn die Rastvorrichtung (48, 108) nicht gelöst ist, wobei bevorzugt die Rastvorrichtung (48, 108) am oder im Pumpkolben (2, 62) angeordnet ist oder sich durch den Pumpkolben (2, 62) erstreckt.

15. Verfahren zum Mischen eines Knochenzements, insbesondere mit einer Vorrichtung nach einem der Ansprüche 1 bis 14, mit den chronologischen Schritten
A) ein Pumpkolben (2, 62) wird in einen Hohlzylinder (1, 61) gedrückt, wobei durch die Bewegung des Pumpkolbens (2, 62) eine Öffnungseinrichtung (3, 63) gegen eine verformbare Seitenwand (6, 66) einer Aufnahme (4, 64) gedrückt wird,
B) durch die Verformung der Seitenwand (6, 66) der Aufnahme (4, 64) mit der Öffnungseinrichtung (3, 63) wird in der Aufnahme (4, 64) ein Monomerflüssigkeitsbehälter (5, 65) enthaltend eine Monomerflüssigkeit geöffnet,
C) die Monomerflüssigkeit fließt aus dem geöffneten Monomerflüssigkeitsbehälter (5, 65) aus und in den Hohlzylinder (1, 61) hinein,
D) der Pumpkolben (2, 62) wird weiter in den Hohlzylinder (1, 61) gedrückt und die Monomerflüssigkeit dadurch aus dem Hohlzylinder (1, 61) und durch eine Verbindungsleitung (9, 69) in den Innenraum einer Kartusche (8, 68) eingepresst, wobei sich in dem Innenraum der Kartusche (8, 68) ein Zementpulver befindet, und
E) die Monomerflüssigkeit und das Zementpulver werden in dem Innenraum der Kartusche (8, 68) gemischt.

16. Verfahren nach Anspruch 15, **dadurch gekennzeichnet, dass** in Schritt A) die Bewegung des Pumpkolbens (2, 62) durch eine Rastvorrichtung (48, 108) gestoppt wird und vor Schritt D) die Rastvorrichtung (48, 108) gelöst wird, um den Pumpkolben (2, 62) weiter in den Hohlzylinder (1, 61) drücken zu können.

17. Verfahren nach Anspruch 15 oder 16, **dadurch gekennzeichnet, dass** der Pumpkolben (2, 62) von einem gespannten Federelement (75) in den Hohlzylinder (1, 61) gedrückt wird, wobei hierfür vorzugsweise zuvor ein Rastmittel (71) oder eine Arretierung gelöst wird, die in den Pumpkolben (2, 62) und/oder in das Federelement (75) greift.

18. Verfahren nach einem der Ansprüche 15 bis 17, **dadurch gekennzeichnet, dass** die Kartusche (8, 68) mit dem fertig gemischten Zementteig von der Verbindungsleitung (9, 69), dem Hohlzylinder (1, 61) und der Aufnahme (4, 64) gelöst wird und der fertig gemischte Zementteig durch Vortreiben eines Austragskolbens (26, 28, 86, 88), der axial beweglich in der Kartusche (8, 68) gelagert ist und der den Innenraum der Kartusche (8, 68) auf einer Seite begrenzt, aus dem Innenraum der Kartusche (8, 68) ausgetragen wird.

19. Verfahren nach einem der Ansprüche 15 bis 18, **dadurch gekennzeichnet, dass** die Monomerflüssigkeit durch ein Vakuum im Innenraum der Kartusche (8, 68) in die Kartusche (8, 68) gesaugt wird, wobei vorzugsweise das Vakuum in der Kartusche (8, 68) durch Evakuieren des Innenraums der Kartusche (8, 68) durch den an der Kartusche (8, 68) arretierten Austragskolben (26, 28, 86, 88) erfolgt.

## Claims

1. Device for the mixing of polymethylmethacrylate bone cement and for storage of the starting components of the bone cement, in particular of a monomer liquid and a cement powder as starting components of the bone cement, the device comprising
a cartridge (8, 68) with an internal space for the mixing of the bone cement, whereby the internal space is closed on one side by a mobile dispensing plunger (26, 28, 86, 88);
a receptacle (4, 64) with side walls, at least regions of which are closed, for accommodation of a monomer liquid container (5, 65), whereby the receptacle (4, 64) comprises at least one deformable closed side wall (6, 66);
a sieve (42, 102) and/or a filter (42, 102) that is or are arranged below the receptacle (4, 64) such that the content of the opened monomer liquid container (5, 65) flows through the sieve (42, 102) and/or the filter (42, 102); a connecting conduit (9, 69) through which the monomer liquid can be conducted into the internal space of the cartridge (8, 68);
a hollow cylinder (1, 61) that is connected to the connecting conduit (9, 69) and is connected to the receptacle (4, 64) by means of a fluid connection (44, 104) such that the hollow cylinder (1, 61) is arranged in a fluid conduit (9, 44, 69, 104) between the receptacle (4, 64) and the internal space of the cartridge (8, 68), whereby a pumping plunger (2, 62) that can be shifted axially in the hollow cylinder (1, 61) is arranged in the hollow cylinder (1, 61); and
an opening facility (3, 63) that is supported, such as to be mobile, against the deformable side wall (6, 66) of the receptacle (4, 64), whereby the opening facility (3, 63) extends all the way into the hollow cylinder (1, 61), whereby the opening facility (3, 63) can be pushed against the deformable side wall (6, 66) of the receptacle (4, 64) by pushing the pumping plunger (2, 62) into the hollow cylinder (1, 61), such that the deformable side wall (6, 66) deforms appropriately such that a monomer liquid container (5, 65) situated in the receptacle (4, 64) can be opened by means of the pressure of the opening facility (3, 63).

2. Device according to claim 1, **characterised in that**
the monomer liquid can flow from the opened monomer liquid container (5, 65) into the hollow cylinder (1, 61), and the connecting conduit (9, 69) appropriately connects the hollow cylinder (1, 61) to the internal space of the cartridge (8, 68) such that the pumping plunger (2, 62) can be used to push monomer liquid from the hollow cylinder (1, 61) through the connecting conduit (9, 69) into the internal space of the cartridge (8, 68) by pushing the pumping plunger (2, 62) into the hollow cylinder (1, 61).

3. Device according to any one of the preceding claims, **characterised in that** a filter (18, 78) that is impermeable to the cement powder and is permeable to the monomer liquid is arranged between the connecting conduit (9, 69) and the internal space of the cartridge (8, 68).

4. Device according to any one of the preceding claims, **characterised in that** the device comprises a pedestal (38, 40, 98, 100), in which at least a part of the connecting conduit (9, 69) is arranged, whereby the cartridge (8, 68) is detachably connected to the pedestal (38, 40, 98, 100), in particular is detachably connected to the pedestal (38, 40, 98, 100) by means of a screw thread (14, 16, 74, 76), whereby, if applicable, the filter (18, 78) that is impermeable to the cement powder and is permeable to the monomer liquid is preferred to be arranged in the pedestal (38, 40, 98, 100) of the device, particularly preferably is arranged in the connection (12, 72) of the cartridge (8, 68) of the pedestal (38, 40, 98, 100).

5. Device according to any one of the preceding claims, **characterised in that** the receptacle (4, 64) merges into the hollow cylinder (1, 61) on a jacket surface of the hollow cylinder (1, 61).

6. Device according to any one of the preceding claims, **characterised in that** the hollow cylinder (1,61) comprises, on the side opposite from the pumping plunger (2, 62), a conical, semi-spherical or other floor (50, 110) that tapers in downward direction, whereby the surface (52, 112) of the pumping plunger (2, 62) facing the floor (50, 110) of the hollow cylinder (1,61) forms a negative mould of the floor (50, 110).

7. Device according to any one of the preceding claims, **characterised in that** the device is a tensioned compression spring (75) and comprises at least one locking mechanism (48, 71, 108), whereby the compression spring (75) and/or the pumping plunger (2, 62) is or are detachably locked to the locking mechanism (48, 71, 108), whereby the compression spring (75) exerts a pressure onto the pumping plunger (2, 62) when the locking mechanism (48, 71, 108) is unlocked, such that the pumping plunger (2, 62) is being pressed into the hollow cylinder (1, 61).

8. Device according to any one of the preceding claims, **characterised in that** the connecting conduit (9, 69) comprises an upward facing loop (10, 70) between the hollow cylinder (1,61) and the internal space of the cartridge (8, 68), whereby the apex of the loop (10, 70) is situated above the merging site (44, 104) of the receptacle (4, 64) into the hollow cylinder (1, 61).

9. Device according to any one of the preceding claims, **characterised in that** the volume in the hollow cylinder (1, 61) is smaller than or equal to the volume of the monomer liquid in the monomer liquid container (5, 65).

10. Device according to any one of the preceding claims, **characterised in that** the opening facility (3, 63) is a lever (3, 63) that is supported against the receptacle (4, 64) such that it can rotate about an axis, whereby a free end (7, 67) of the lever (3, 63) can be pushed against the deformable side wall (6, 66) of the receptacle (4, 64) by the pumping plunger (2, 62), such that the free end (7, 67) of the lever (3, 63) appropriately deforms the deformable side wall (6, 66) such that a monomer liquid container (5, 65) that is situated in the receptacle (4, 64) and fits the receptacle (4, 64) can be opened by the pressure of the free end (7, 67) of the lever (3, 63), preferably can be broken open or slit open or punctured open.

11. Device according to any one of the preceding claims, **characterised in that** the receptacle (4, 64) is a hollow cylinder (1, 61) and/or the receptacle (4, 64) consists of an elastomer or comprises an insert made of an elastomer, whereby the elastomer preferably comprises a Shore hardness of more than 60, whereby the elastomer particularly preferably is a silicone rubber or an ethylene-propylene-diene rubber (EPDM).

12. Device according to any one of the preceding claims, **characterised in that** the receptacle (4, 64) has a ledge for supporting the monomer liquid container (5, 65) arranged on it, whereby the ledge is smaller than half of the surface of the floor of the monomer liquid container (5, 65) or of the cross-section of the monomer liquid container (5, 65).

13. Device according to any one of the preceding claims, **characterised in that**, upon a motion of the pumping plunger (2, 62) into the hollow cylinder (1, 61), a free end (7, 67) of the opening facility (3, 63) appropriately pushes onto the deformable side wall (6, 66) such that the force vector comprises a component that is directed in the direction of the sieve (42, 102) and/or filter (42, 102) and/or pushes a monomer liquid container (5, 65) that is inserted into the receptacle (4, 64) into the receptacle (4, 64), preferably in the direction of the ledge.

14. Device according to any one of the preceding claims, **characterised in that** the pumping plunger (2, 62) can be fixed in place by means of a manually detachable snap-in device (48, 108) which, after axial shifting of the pumping plunger (2, 62) over the opening facility (3, 63) in the hollow cylinder (1, 61), blocks the pumping plunger (2, 62) above the merging site of the fluid connection (44, 104) to the receptacle (4, 64) in the hollow cylinder (1, 61), such that the pumping plunger (2, 62) cannot be moved over the merging site of the fluid connection (44, 104) in the hollow cylinder (1, 61), when the snap-in device (48, 108) is not detached, whereby the snap-in device (48, 108) is preferably arranged on or in the pumping plunger (2, 62) or extends through the pumping plunger (2, 62).

15. Method for the mixing of a bone cement, in particular through the use of a device according to any one of the claims 1 to 14, comprising the chronological steps of
A) pushing a pumping plunger (2, 62) into a hollow cylinder (1, 61), whereby the motion of the pumping plunger (2, 62) pushes an opening facility (3, 63) against a deformable side wall (6, 66) of a receptacle (4, 64);
B) the deformation of the side wall (6, 66) of the receptacle (4, 64) by the opening facility (3, 63) opens a monomer liquid container (5, 65) containing a monomer liquid in the receptacle (4, 64);
C) the monomer liquid flows out of the opened monomer liquid container (5, 65) and into the hollow cylinder (1, 61);
D) further pushing the pumping plunger (2, 62) into the hollow cylinder (1, 61) and, thus, the monomer liquid is pressed out of the hollow cylinder (1, 61) and through a connecting conduit (9, 69) into the internal space of a cartridge (8, 68), whereby a cement powder is situated in the internal space of the cartridge (8, 68); and
E) the monomer liquid and the cement powder are being mixed in the internal space of the cartridge (8, 68).

16. Method according to claim 15, **characterised in that** the motion of the pumping plunger (2, 62) in step A) is stopped by a snap-in device (48, 108), and the snap-in device (48, 108) is being detached before step D) in order to be able to further push the pumping plunger (2, 62) into the hollow cylinder (1, 61).

17. Method according to claim 15 or 16, **characterised in that** the pumping plunger (2, 62) is being pushed into the hollow cylinder (1, 61) by a tensioned spring element (75), whereby a snap-in means (71) or a locking mechanism engaging the pumping plunger (2, 62) and/or the spring element (75) is preferably detached first for this purpose.

18. Method according to any one of the claims 15 to 17, **characterised in that** the cartridge (8, 68) with the ready-mixed cement dough is being detached from the connecting conduit (9, 69), the hollow cylinder (1, 61), and the receptacle (4, 64) and the ready-mixed cement dough is being dispensed from the internal space of the cartridge (8, 68) by propelling a dispensing plunger (26, 28, 86, 88) that is supported such as to be axially mobile in the cartridge (8, 68) and that bounds the internal space of the cartridge (8, 68) on one side.

19. Method according to any one of the claims 15 to 18, **characterised in that** the monomer liquid is being aspirated into the cartridge (8, 68) by a vacuum in the internal space of the cartridge (8, 68), whereby the vacuum in the cartridge (8, 68) preferably is produced by evacuating the internal space of the cartridge (8, 68) through the dispensing plunger (26, 28, 86, 88) that is locked onto the cartridge (8, 68).

## Revendications

1. Dispositif pour le mélange de ciment osseux de polyméthacrylate de méthyle et pour le stockage des composants initiaux du ciment osseux, notamment d'un liquide monomère et d'une poudre de ciment comme composants initiaux du ciment osseux, le dispositif présentant
une cartouche (8, 68) avec un espace intérieur pour le mélange du ciment osseux, l'espace intérieur étant obturé sur un côté par un piston d'extraction (26, 28, 86, 88) mobile,
une prise (4, 64) avec des parois latérales fermées au moins partiellement pour la réception d'un réservoir de liquide monomère (5, 65), la prise (4, 64) présentant au moins une paroi latérale (6, 66) fermée, déformable,
un tamis (42, 102) et/ou un filtre (42, 102) qui est disposé ou sont disposés en dessous de la prise (4, 64) de façon à ce que le contenu du réservoir de liquide monomère (5, 65) ouvert coule à travers le tamis (42, 102) et/ou le filtre (42, 102),
une ligne de raccordement (9, 69) par laquelle le liquide monomère est à acheminer dans l'espace intérieur de la cartouche (8, 68),
un cylindre creux (1, 61) qui est relié à la ligne de raccordement (9, 69) et qui est relié à la prise (4, 64) par une liaison fluidique (44, 104) de façon à ce que le cylindre creux (1, 61) soit disposé dans une ligne fluidique (9, 44, 69, 104) entre la prise (4, 64) et l'espace intérieur de la cartouche (8, 68), un piston de pompe (2, 62) déplaçable en sens axial dans le cylindre creux (1, 61) étant disposé dans le cylindre creux (1, 61) et
un dispositif d'ouverture (3, 63) qui est supporté de manière mobile contre la paroi latérale (6, 66) déformable de la prise (4, 64), le dispositif d'ouverture (3, 63) s'étendant jusqu'au cylindre creux (1, 61), le dispositif d'ouverture (3, 63) étant à appuyer par pression du piston de pompe (2, 62) dans le cylindre creux (1, 61) contre la paroi latérale (6, 66) déformable de la prise (4, 64) de telle sorte que la paroi latérale (6, 66) déformable soit déformée de manière à ce qu'un réservoir de liquide monomère (5, 65) se trouvant dans la prise (4, 64) puisse être ouvert par la pression du dispositif d'ouverture (3, 63).

2. Dispositif conformément à la revendication n°1, **caractérisé par le fait que** le liquide monomère provenant du réservoir de liquide monomère (5, 65) ouvert peut couler dans le cylindre creux (1, 61) et que la ligne de raccordement (9, 69) relie le cylindre creux (1, 61) à l'espace intérieur de la cartouche (8, 68) de manière à ce que du liquide monomère soit à presser du cylindre creux (1, 61) avec le piston de pompe (2, 62) par pression du piston de pompe (2, 62) dans le cylindre creux (1, 61) par la ligne de raccordement (9, 69) dans l'espace intérieur de la cartouche (8, 68).

3. Dispositif conformément à l'une des revendications précédentes, **caractérisé par le fait que**
un filtre (18, 78) imperméable à la poudre de ciment et perméable au liquide monomère est disposé entre la ligne de raccordement (9, 69) et l'espace intérieur de la cartouche (8, 68).

4. Dispositif conformément à l'une des revendications précédentes, **caractérisé par le fait que**
le dispositif présente un pied de support (38, 40, 98, 100) dans lequel est disposée au moins une partie de la ligne de raccordement (9, 69), la cartouche (8, 68) étant reliée de manière amovible au pied de support (38, 40, 98, 100), étant en particulier reliée par un pas de vis (14, 16, 74, 76) au pied de support (38, 40, 98, 100), le filtre (18, 78) imperméable à la poudre de ciment et perméable au liquide monomère étant, le cas échéant, de préférence disposé dans le pied de support (38, 40, 98, 100) du dispositif, étant encore plus préférablement disposé dans la ligne (12, 72) vers la cartouche (8, 68) du pied de support (38, 40, 98, 100).

5. Dispositif conformément à l'une des revendications précédentes, **caractérisé par le fait que**
la prise (4, 64) conduise à une surface enveloppante du cylindre creux (1, 61) dans le cylindre creux (1, 61).

6. Dispositif conformément à l'une des revendications précédentes, **caractérisé par le fait que**
le cylindre creux (1, 61) présente, sur le côté opposé au piston de pompe (2, 62), un fond (50, 110) conique, hémisphérique ou autre, s'effilant vers le bas, la surface (52, 112) du piston de pompe (2, 62), tournée vers le fond (50, 110) du cylindre creux (1, 61), formant une forme négative du fond (50, 110).

7. Dispositif conformément à l'une des revendications précédentes, **caractérisé par le fait que**
le dispositif présente un ressort de compression (75) tendu et au moins un système d'arrêt (48, 71, 108), le ressort de compression (75) et/ou le piston de pompe (2, 62) étant bloqué(s) de manière amovible avec le système d'arrêt (48, 71, 108), le ressort de compression (75) exerçant une pression sur le piston de pompe (2, 62) quand le système d'arrêt (48, 71, 108) est desserré de sorte que le piston de pompe (2, 62) soit comprimé dans le cylindre creux (1, 61).

8. Dispositif conformément à l'une des revendications précédentes, **caractérisé par le fait que**
la ligne de raccordement (9, 69) entre le cylindre creux (1, 61) et l'espace intérieur de la cartouche (8, 68) présente une boucle (10, 70) tournée vers le haut, le point le plus haut de la boucle (10, 70) se situant au-dessus de la jonction (44, 104) de la prise (4, 64) dans le cylindre creux (1, 61).

9. Dispositif conformément à l'une des revendications précédentes, **caractérisé par le fait que**
le volume dans le cylindre creux (1, 61) est inférieur ou égal au volume du liquide monomère dans le réservoir de liquide monomère (5, 65).

10. Dispositif conformément à l'une des revendications précédentes, **caractérisé par le fait que**
le dispositif d'ouverture (3, 63) est un levier (3, 63) qui est monté de façon tournante autour d'un axe contre la prise (4, 64), une extrémité libre (7, 67) du levier (3, 63) pouvant être pressée depuis le piston de pompe (2, 62) contre la paroi latérale (6, 66) déformable de la prise (4, 64) de façon à ce que l'extrémité libre (7, 67) du levier (3, 63) déforme la paroi latérale (6, 66) déformable de telle manière à ce qu'un réservoir de liquide monomère (5, 65) se trouvant dans la prise (4, 64) et approprié à la prise (4, 64) puisse être ouvert, en particulier rompu ou entaillé ou percé par la pression de l'extrémité libre (7, 67) du levier (3, 63).

11. Dispositif conformément à l'une des revendications précédentes, **caractérisé par le fait que**
la prise (4, 64) est un cylindre creux (1, 61) et/ou que la prise (4, 64) se compose d'un élastomère ou comprend une garniture composée d'un élastomère, l'élastomère présentant, de préférence, une dureté Shore supérieure à 60, l'élastomère étant, encore plus préférablement, un caoutchouc de silicone ou un caoutchouc éthylène-propylène-diène (EPDM).

12. Dispositif conformément à l'une des revendications précédentes, **caractérisé par le fait que**
un épaulement pour le support du réservoir de liquide monomère (5, 65) est disposé dans la prise (4, 64), l'épaulement étant plus petit que la moitié de la surface du fond du réservoir de liquide monomère (5, 65) ou de la coupe transversale du réservoir de liquide monomère (5, 65).

13. Dispositif conformément à l'une des revendications précédentes, **caractérisé par le fait que**
une extrémité libre (7, 67) du dispositif d'ouverture (3, 63) appuie sur la paroi latérale (6, 66) déformable lors d'un mouvement du piston de pompe (2, 62) dans le cylindre creux (1, 61) de telle manière à ce que le vecteur de la force présente un composant qui est dirigé vers le tamis (42, 102) et/ou le filtre (42, 102) et/ou qui enfonce dans la prise (4, 64) un réservoir de liquide monomère (5, 65) utilisé dans la prise (4, 64), de préférence en direction de l'épaulement.

14. Dispositif conformément à l'une des revendications précédentes, **caractérisé par le fait que**
le piston de pompe (2, 62) est fixable avec un dispositif d'encliquetage (48, 108) desserrable manuellement, qui bloque le piston de pompe (2, 62) au-dessus de la jonction de la liaison fluidique (44, 104) avec la prise (4, 64) dans le cylindre creux (1, 61) après déplacement axial du piston de pompe (2, 62) au-dessus du dispositif d'ouverture (3, 63) dans le cylindre creux (1, 61) de telle manière à ce que le piston de pompe (2, 62) dans le cylindre creux (1, 61) ne soit pas déplaçable au-dessus de la jonction de la liaison fluidique (44, 104) si le dispositif d'encliquetage (48, 108) n'est pas desserré, le dispositif d'encliquetage (48, 108) étant de préférence disposé sur ou dans le piston de pompe (2, 62) ou s'étendant par le piston de pompe (2, 62).

15. Procédé pour le mélange d'un ciment osseux, en particulier avec un dispositif conformément à l'une des revendications n°1 à n°14, avec les étapes chronologiques
A) un piston de pompe (2, 62) est enfoncé dans un cylindre creux (1, 61), un dispositif d'ouverture (3, 63) étant appuyé contre une paroi latérale (6, 66) déformable d'une prise (4, 64) par le mouvement du piston de pompe (2, 62)
B) un réservoir de liquide monomère (5, 65) contenant un liquide monomère est ouvert dans la prise (4, 64) par la déformation de la paroi latérale (6, 66) de la prise (4, 64) avec le dispositif d'ouverture (3, 63),
C) le liquide monomère s'écoule du réservoir de liquide monomère (5, 65) ouvert et entre dans le cylindre creux (1, 61),
D) le piston de pompe (2, 62) est davantage enfoncé dans le cylindre creux (1, 61) et le liquide monomère est ainsi pressé depuis le cylindre creux (1, 61) et par une ligne de raccordement (9, 69) dans l'espace intérieur d'une cartouche (8, 68), une poudre de ciment se trouvant dans l'espace intérieur de la cartouche (8, 68) et
E) le liquide monomère et la poudre de ciment sont mélangés dans l'espace intérieur de la cartouche (8, 68).

16. Procédé conformément à la revendication n°15, **caractérisé par le fait que** dans l'étape A), le mouvement du piston de pompe (2, 62) est arrêté par un dispositif d'encliquetage (48, 108) et, avant l'étape D), le dispositif d'encliquetage (48, 108) est desserré pour pouvoir enfoncer davantage le piston de pompe (2, 62) dans le cylindre creux (1, 61).

17. Procédé conformément à la revendication n°15 ou n°16, **caractérisé par le fait que**
le piston de pompe (2, 62) est enfoncé par un élément élastique (75) tendu dans le cylindre creux (1, 61), un moyen d'encliquetage (71) ou un système d'arrêt qui entre dans le piston de pompe (2, 62) et/ou dans l'élément élastique (75), étant de préférence préalablement desserré à cet effet.

18. Procédé conformément à l'une des revendications n°15 à n°17, **caractérisé par le fait que**
la cartouche (8, 68) avec la pâte de ciment mélangée est desserrée de la ligne de raccordement (9, 69), du cylindre creux (1, 61) et de la prise (4, 64) et que la pâte de ciment mélangée est évacuée de l'espace intérieur de la cartouche (8, 68) par la propulsion d'un piston d'extraction (26, 28, 86, 88) qui est monté de façon mobile en sens axial dans la cartouche (8, 68) et qui limite l'espace intérieur de la cartouche (8, 68) d'un côté.

19. Procédé conformément à l'une des revendications n°15 à n°18, **caractérisé par le fait que**
le liquide monomère est aspiré dans la cartouche (8, 68) par un vide dans l'espace intérieur de la cartouche (8, 68), le vide opérant de préférence dans la cartouche (8, 68) par l'évacuation de l'espace intérieur de la cartouche (8, 68) par le piston d'extraction (26, 28, 86, 88) bloqué sur la cartouche (8, 68).
